# EUROPÄISCHE PATENTSCHRIFT

(11) **EP 1 313 870 B1**
(45) Veröffentlichungstag und Bekanntmachung des Hinweises auf die Patenterteilung: **21.09.2005**
(21) Anmeldenummer: 01965232.0
(22) Anmeldetag: 30.08.2001
(51) Int. Cl.: C12P 7/42, C12N 1/21, C07C 13/23, C12N 9/02, C12N 9/14, C12N 9/90

(54) **VERFAHREN ZUR VERBESSERTEN HERSTELLUNG UND ISOLIERUNG VON TRANS-DIHYDROXY-CYCLOHEXADIEN-CARBONSÄUREN UND/ODER DEREN FOLGEPRODUKTE SOWIE EIN DAZU GEEIGNETER GENETISCH VERÄNDERTER ORGANISMUS**
METHOD FOR THE IMPROVED PRODUCTION AND ISOLATION OF TRANS-DIHYDROXYCYCLOHEXADIENE CARBOXYLIC ACIDS AND/OR DERIVATIVES THEREOF AND A GENETICALLY MODIFIED ORGANISM SUITABLE FOR THE ABOVE
PROCEDE PERMETTANT D'AMELIORER LA PREPARATION ET L'ISOLATION D'ACIDES CARBOXYLIQUES I TRANS /I -DIHYDROXYCYCLOHEXADIENE ET/OU DE LEURS DERIVES, ET ORGANISME GENETIQUEMENT MODIFIE APPROPRIE

(30) Priorität: 30.08.2000 DE 10042535
(43) Veröffentlichungstag der Anmeldung: 28.05.2003
(73) Patentinhaber: Forschungszentrum Jülich, 52456 Jülich (DE)
(72) Erfinder: FRANKE, Dirk, 54597 Seiwerath (DE); SPRENGER, Georg, 52428 Jülich (DE); LORBACH, Volker, 52353 Düren (DE); TAKORS, Ralf, 52399 Merzenich (DE); MÜLLER, Michael, 52428 Jülich (DE); HALFAR, Markus, 52428 Jülich (DE); MÜLLER, Rolf, 38302 Wolfenbüttel (DE); THÖMMES, Jörg, San Diego, CA 92130 (US)
(74) Vertreter: Fitzner, Uwe
(86) Internationale Anmeldenummer: PCT/EP2001/009980
(87) Internationale Veröffentlichungsnummer: WO 2002/018611

(56) Entgegenhaltungen:
- MUELLER ROLF ET AL: "Bacterial production of trans-dihydroxycyclohexadiene carboxylates by metabolic pathway engineering." MICROBIOLOGY (READING), Bd. 142, Nr. 4, 1996, Seiten 1005-1012, XP008003257 ISSN: 1350-0872 in der Anmeldung erwähnt
- GRISOSTOMI CORINNA ET AL: "Efficient in vivo synthesis and rapid purification of chorismic acid using an engineered Escherichia coli strain." BIOORGANIC CHEMISTRY, Bd. 25, Nr. 5-6, 1997, Seiten 297-305, XP002199067 ISSN: 0045-2068 in der Anmeldung erwähnt
- RUSNAK F ET AL: "SUBCLONING OF THE ENTEROBACTIN BIOSYNTHETIC GENE ENT-B EXPRESSION PURIFICATION CHARACTERIZATION AND SUBSTRATE SPECIFICITY OF ISOCHORISMATASE" BIOCHEMISTRY, Bd. 29, Nr. 6, 1990, Seiten 1425-1435, XP002199068 ISSN: 0006-2960 in der Anmeldung erwähnt
- SCHMIDT KARSTEN ET AL: "Microbial production of (+)-trans-isochorismic acid." BIOTECHNOLOGY AND BIOENGINEERING, Bd. 45, Nr. 4, 1995, Seiten 285-291, XP001077012 ISSN: 0006-3592 in der Anmeldung erwähnt

## Beschreibung

Die vorliegende Erfindung betrifft ein Verfahren zur verbesserten Herstellung und Isolierung von *trans*-Dihydroxy-Cyclohexadien-Carbonsäuren und/oder deren Folgeprodukte sowie einen dazu geeigneten genetisch veränderten nicht-pathogenen Organismus und die Verwendung der *trans*-DCHCs.

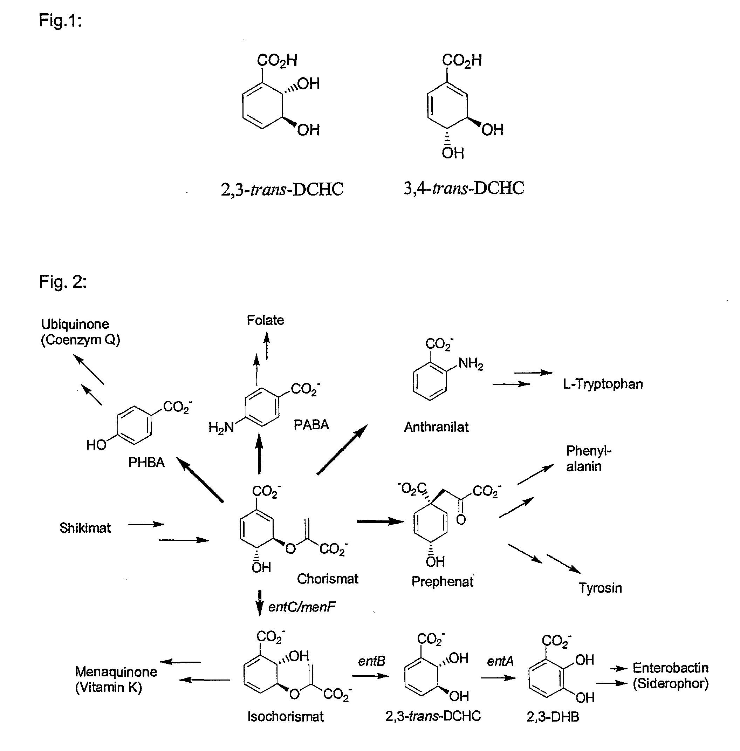

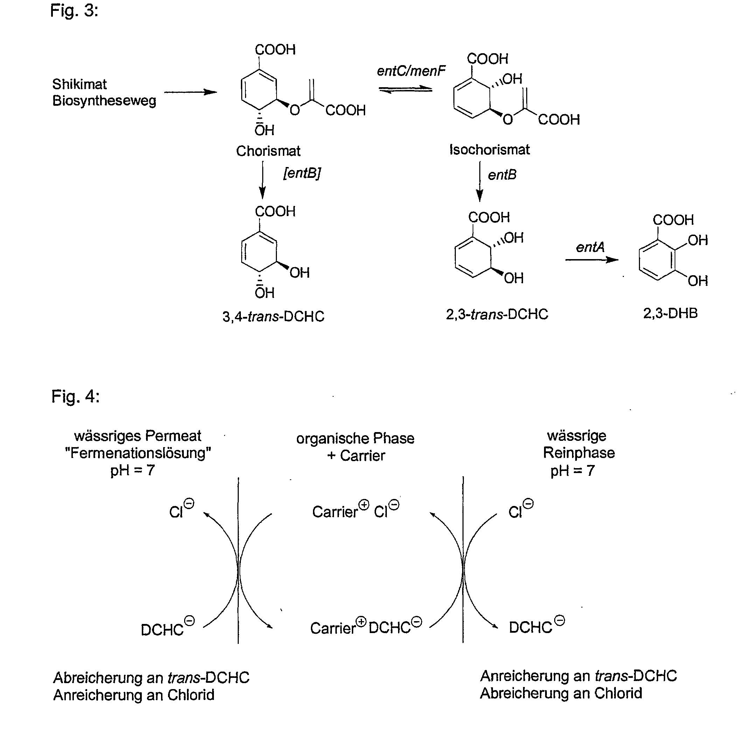

*Trans*-Dihydroxy-Cyclohexadien-Carbonsäure (*trans*-DCHCs) stellen im Rahmen des Shikimisäure-Biosyntheseweges (Fig.2) die Folgemetabolite des Chorismats dar. Bei den *trans*-DCHCs unterscheidet man zwischen zwei Konstitutionsisomeren (Stereoisomeren), nämlich 2,3-*trans*-DCHC (entsprechend (5*S*,6*S*)-Dihydroxy-cyclohexa-1,3-diencarbonsäure) und 3,4-*trans*-DCHC (entsprechend (3*R*,4*R*)-Dihydroxy-cyclohexa1,5-diencarbonsäure), wie in Fig. 1 dargestellt. Ausgehend von Chorismat entsteht über intermediäres Isochorismat das 2,3-*trans*-DCHC. Die Transformation erfolgt über zwei Enzyme: Isochorismatsynthase (kodiert durch das Gen *entC*) und Isochorismatase (kodiert durch das Gen *entB*). 2,3-*trans*-DCHC ist ein Zwischenprodukt auf dem Biosyntheseweg des Enterobactin, welches als Chelatbildner im Fall von Eisenmangel in der Zelle gebildet und sekretiert wird. Das für den Abbau von 2,3-*trans*-DCHC verantwortliche Enzym ist die 2,3-Dihydroxybenzoatsynthase (kodiert durch das Gen *entA*). Eine Übersicht ist in Fig. 3 dargestellt. 3,4-*trans*-DCHC konnte bislang in Prokaryonten nicht beobachtet werden. Nach bisherigem Kenntnisstand entsteht es in kleineren Mengen bei Überexpression von *entB*, besitzt jedoch keine biologische Funktion.

Die Herstellung von Metaboliten des Aromatenbiosyntheseweges und insbesondere von *trans*-Dihydroxy-Cyclohexadien-Carbonsäuren (*trans*-DCHC) sowohl auf enzymatisch-fermentativem als auch auf rein chemischem Wege ist bereits beschrieben. Die chemische Synthese ist dabei sehr aufwendig und mit zahlreichen in der Literatur beschriebenen Nachteilen behaftet. Kennzeichnend für die chemischen Synthesen ist, daß sie keine oder nur eine sehr geringe Enantioselektivität aufweisen und aufgrund der sehr hohen Aromatisierungs-Empfindlichkeit der *trans*-DCHC mit schlechten Ausbeuten verbunden sind. Außerdem sind zur chemischen Herstellung vielstufige Synthesesequenzen und meist hohe Reaktionstemperaturen mit hohem Energieeintrag notwendig. Darüber hinaus sind die chemischen Synthesen durch die Verwendung giftiger, explosiver oder umweltgefährdender Substanzen und die erforderlichen Sicherheits- und Entsorgungsmaßnahmen unökonomisch. Nachfolgend wird deshalb insbesondere auf die enzymatisch-fermentative Herstellung eingegangen. Hierbei sind zur Herstellung von *trans*-Dihydroxy-Cyclohexadien-Carbonsäuren sowohl *in-vitro-* als auch *in-vivo*-Verfahren bekannt.

So beschreiben Rusnak, F., et al. (Biochemistry, 1990, 29: 1425-1435 und Liu, J. et al. (Biochemistry, 1990, 29:1417-1425) generell NMR-Messungen zur *in-vitro*-Umsetzung von Isochorismat zu 2,3-*trans*-DCHC mit gereinigter Isochorismatsynthase (EntC) bzw. Isochorismatase (EntB). Die *in-vitro*-Herstellung von *trans*-DCHC ausgehend von Chorismat mit Hilfe von Zellextrakten aus *E. coli*-Stämmen, die entweder eine Mutation in den Genen *entA, entB* oder *entC* aufwiesen ist bei Young, I. G. et al. (J. Bacteriology, 1971, 106: 51-57) beschrieben. Die *in-vitro* Umsetzung von vorgelegtem Chorismat zu 2,3-*trans*-DCHC mittels eines Zellextraktes aus Klebsiella-Stämmen beschreiben Young, I. G. et al. (Biochimica et Biophysica Acta, 1969, 177: 381-388 und Biochimica et Biophysica Acta, 1969, 177:401-411). Nachteilig bei den hier beschrieben Verfahren ist jedoch die sehr kurze Haltbarkeit der verwendeten Zellaufschlüsse. Weitaus nachteiliger ist jedoch, daß Chorismat als Ausgangssubstrat nur in geringen Mengen verfügbar und zudem sehr teuer ist (ca. 1.400,-DM/Gramm).

Eine enzymatisch-fermentative Synthese von Chorismat, als Vorstufe der *trans*-DCHC, mit Hilfe eines lebenden Systems, bei dem die verwendeten *E. coli*-Stämme Auxotrophien für die aromatischen Aminosäuren aufweisen, ist bei Grisostomi, G. et al. (Bioorganic Chemistry, 1997, 25: 297-305) beschrieben. Allerdings wurden nur geringe Mengen an Chorismat (520 mg/l) produziert. Aufgrund der Instabilität des Chorismats ist eine weitere Aufarbeitung von hohen Verlusten gekennzeichnet.
Die *in-vivo*-Produktion einer weiteren Vorstufe von *trans*-DCHC, nämlich von Isochorismat mit *Klebsiella pneumoniae* ist bei Schmidt, K. et al. (Biotechnol. Bioeng. 1995, 45: 285-291) beschrieben. Hierbei entstehen lediglich 90 mg/l Isochorismat. Isochorismat weist eine noch größere Instabilität auf als Chorismat, so daß auch dieses Verfahren zur Bereitstellung größerer Mengen an Vorstufen bzw. Bausteinen zur Herstellung von *trans*-DCHC und deren Folgeprodukte ungeeignet ist.

Bei Müller, R. et al. (Microbiology, 1996, 142: 1005-1012) wird die Exkretion von *trans*-DCHC durch ein *in-vivo*-System, nämlich aus einem potentiell human-pathogenen *Klebsiella pneumoniae*-Stamm beschrieben. Diese Stämme wiesen eine Anzahl verschiedener Nachteile auf. So zeigen die *Klebsiella-pneumoniae*-Stämme aufgrund verschiedener Punktmutationen Auxotrophien für die drei aromatischen Aminosäuren Phenylalanin, Tyrosin und Tryptophan, so daß diese essentiellen Aminosäuren dem Kulturmedium zugesetzt werden müssen. Der Organismus neigt ferner aufgrund der Punktmutationen relativ schnell zu spontanen Rückmutationen, ist aufgrund dessen genetisch relativ instabil und für großtechnische Fermentationsprozesse wenig geeignet. Zusätzlich enthielten die Klebsiella-Stämme heterologe Gene aus *E. coli* und zwar die Gene *entB*/*entC* oder *entB* alleine (Gibson, M. I. et al.; Biochem. J., 1964, 90: 248-256). Eine Veränderung des *entA*-Gens ist für diese Stämme nicht offenbart. Nachteilig bei dem hier beschriebenen Verfahren ist insbesondere, daß die gewünschten Produkte *trans*-DCHC nicht exklusiv entstehen, sondern stark mit diversen Metaboliten, insbesondere Chorismat verunreinigt sind. Neben Chorismat konnten hier etwa 90 mg/l 2,3-*trans*-DCHC bei Überexpression von *entB*/*entC* und etwa 70 mg/l 3,4-*trans*-DCHC bei Überexpression von *entB* aus dem Kulturmedium isoliert werden. Allerdings ist die Isolierung und Aufreinigung solcher Gemische schwierig und erfordert weiteren Zeit- und Kostenaufwand, verbunden mit weiteren Verlusten an gewünschtem Produkt. Darüber hinaus ist das Bakterium *Klebsiella pneumoniae* pathogen, d.h. potentiell humanpathogen und den Sicherheitsanforderungen der Risikogruppe 2 unterlegen. Der industrielle Einsatz von pathogenen oder potentiell pathogenen Organismen zur Herstellung biotechnologischer Produkte ist aufgrund hoher produktionstechnischer Sicherheitsauflagen und einer geringen marktwirtschaftlichen Akzeptanz, insbesondere zur Darstellung von Arzneimitteln, äußerst problematisch.

Ferner muß neben der eigentlichen Synthese von trans-DCHC aus Gründen der Wirtschaftlichkeit auch die Isolierung der gewünschten Verbindungen aus dem jeweiligen Produktionsansatz mit zufriedenstellenden Ausbeuten und entsprechender Selektivität gewährleistet sein. Hierbei stellen insbesondere Verunreinigungen des Produktionsansatzes mit ähnlichen Verbindungen, wie z. B. Chorismat, ein Problem dar. Weniger selektive oder "nicht-selektive" Verfahren, wie z. B. Flüssig-Flüssig-Extraktion oder Adsorption/Elution, wie u.a. lonenchromatochraphie sind in diesem Fall nicht geeignet. Ferner schränkt die hohe Empfindlichkeit der *trans*-DCHC gegenüber Säure-/Base- sowie Hitzeeinwirkung die zur Produktaufreinigung in Frage kommenden Separationsverfahren ein.

Daneben stellt die Reaktivextraktion/Reextraktion mit anionischen oder kationischen Carriern ein allgemeines Aufarbeitungsverfahren dar, welches bislang jedoch hauptsächlich zur Trennung von Schwermetallsalzen eingesetzt wird. Beispiele für die Verwendung der Reaktivextraktion zur Produktisolierung in biotechnologischen Prozessen, ebenso wie die Aufkonzentrierung von *trans*-DCHC durch Extraktion sind beschrieben (Müller, R. et al., Microbiology, 1996, 142: 1005-1012). Allerdings sind die Verfahren allesamt darauf ausgelegt, das jeweilige Produkt für analytische Zwecke in hoher Reinheit darzustellen. D. h. zur präparativen Isolierung von *trans*-DCHC aus großtechnischen Produktionsansätzen sind die bislang bekannten Verfahren ungeeignet.

Aufgabe der vorliegenden Erfindung war somit ein verbessertes Verfahren zur Herstellung und Isolierung von *trans*-Dihydroxy-Cyclohexadien-Carbonsäuren zur Verfügung zu stellen, das zufriedenstellende Ausbeuten an reinen *trans*-DCHCs liefert und die zuvor genannten Nachteile nicht mehr aufweist.

Diese Aufgabe wird durch die vorliegende Erfindung in vorteilhafter Weise gelöst.

Gegenstand der vorliegenden Erfindung ist ein Verfahren zur verbesserten Herstellung von *trans*-Dihydroxy-Cyclohexadien-Carbonsäuren und/oder deren Folgeprodukte, wobei die Synthese der *trans*-Dihydroxy-Cyclohexadien-Carbonsäuren in enantiomerenreiner Form ohne nachweisbare Verunreinigungen mit Chorismat ausgehend von erneuerbaren Kohlenstoffquellen durch Kultivierung wenigstens eines genetisch veränderten nicht-pathogenen Organismus erfolgt, welcher über einen Aromatenstoffwechsel verfügt und im Vergleich zu einem entsprechend nicht genetisch veränderten Organismus
a) eine erhöhte Aktivität der Isochorismatsynthase (EntC) und Isochorismatase (EntB) und eine Aktivität der 2,3-Dihydroxybenzoatsynthase (EntA) von nahezu Null aufweist oder
b) eine erhöhte Aktivität der Isochorismatase (EntB) und eine erniedrigte Aktivität der Isochorismatsynthase (EntC) und eine Aktivität der 2,3-Dihydroxybenzoatsynthase (EntA) von nahezu Null aufweist.

Erfindungsgemäß umfaßt sind hierbei auch die in entsprechender Weise veränderten Aktivitäten von Isoenzymen der Isochorismatase und/oder Isochorismatsynthase. Insbesondere umfaßt ist dabei das Isoenzym der Isochorismatsynthase (EntC), welches mit MenF bezeichnet wird und durch das Gen menF kodiert ist (Dahm, C. et al., Biochimica et Biophysica Acta, 1998, 1425: 377-386). Unter Isoenzymen sind Enzyme mit gleicher oder vergleichbarer Substrat- und Wirkungsspezifität zu verstehen, die jedoch eine unterschiedliche Primärstruktur aufweisen. Die Erläuterungen der vorliegenden Erfindung beziehen sich somit gleichermaßen auf EntC und MenF und möglicherweise noch weitere Isoenzyme der Isochorismatsynthase, auch wenn aus Gründen der Klarheit nur EntC explizit genannt wird.

Ein Vorteil des erfindungsgemäßen Verfahrens ist die Herstellung enantiomerenreiner *trans*-DCHC unter Einsatz des zuvor beschriebenen genetisch veränderten und nicht-pathogenen Organismus. Darüber hinaus zeichnet sich das erfindungsgemäße Verfahren dadurch aus, daß die *trans*-Dihydroxy-Cyclohexadien-Carbonsäuren ohne störende Nebenprodukte ähnlicher Struktur, d.h. ohne Verunreinigungen durch Chorismat und/oder Isochorismat, gebildet und aus dem Organismus in ein wässriges Permeat (Kulturmedium) abgegeben werden. Ein weiterer wesentlicher Vorteil der vorliegenden Erfindung ist, daß die Isolierung der *trans*-Dihydroxy-Cyclohexadien-Carbonsäure aus dem wässrigen Permeat durch ein einstufiges Verfahren, d.h. in einem einzigen Schritt erfolgt, ohne zuvor weitere Aufreinigungsschritte, wie z. B. HPLC, durchführen zu müssen. Beispiele für erfindungsgemäß geeignete einstufige Isolierungsverfahren sind die Reaktivextraktion mittels eines kationischen Carriers oder die Adsorption/Desorption an einem Anionenaustauscher oder eine Extraktion (z. B. flüssig-flüssig-Extraktion bei pH 1-3). Hierdurch werden in vorteilhafter Weise erfindungsgemäß hohe Produkt-Reinheiten an enantiomerenreinen *trans*-DCHC von wenigstens 90% erreicht.
Erfindungsgemäß umfaßt sind auch Verfahren, bei denen die Herstellung und Isolierung der gewünschten Produkte nicht nur sukzessive erfolgt, sondern bei denen auch eine integrierte Produktion und Isolierung möglich ist, z.B. im Rahmen kontinuierlich betriebener Fermentationsanlagen bei gleichzeitiger kontinuierlicher Separation der Produkte.

Erfindungsgemäß werden in dem zuvor genannten Verfahren nichtpathogene Organismen, bevorzugt Bakterien, Hefen, Pilze und/oder Pflanzen eingesetzt, die höchstens den Sicherheitsanforderungen der Risikogruppe 1 (definiert durch das Gentechnikgesetz, 1990 BGBI I 1080; 1993 BGBI I 2066; 1993 BGBI III 2121-60-1; 1997 BGBI I 2390) zugeordnet sind und die über einen eigenen Aromaten-Biosyntheseweg verfügen oder in die durch gentechnische Methoden die entsprechenden Informationen eines heterologen Aromaten-Biosyntheseweges eingebracht wurden. Hierunter ist die Einschleusung der Aromaten-Biosynthesegene zu verstehen, die z. B. plasmidkodiert sein können und durch gängige Methoden; wie z. B. Transformation, Konjugation oder Elektroporation übertragen werden und ggf. in das Chromosom der Wirtszellen integriert werden können.
Erfindungsgemäß bevorzugt werden Bakterien und hierbei solche der Gattung der Enterobakterien, insbesondere *Escherichia coli*. Bevorzugte Vertreter können sein: *E. coli* MC4100 (ATCC 35695, DSM 6574) (M. J. Casadaban, *J. Mol. Biol*. 1976, *104,* 541-555 und M. J. Casadaban, S. N. Cohen, *Proc. Natl. Acad. Sci USA* 1979, *76,* 4530-4533 sowie bei Y. Komeda, T. lino, *J. Bacteriol* 1979, *139*, 721-729), *E. coli* H1882, der dem Stamm MC4100 entspricht und sich zusätzlich durch eine Dihydroxybenzoatsynthase-Defzienz (entA-Deletion; Δ(fepA-ent)) auszeichnet oder *E. coli* AN193 (G. B. Cox et al., *J. Bacteriol*. 1970, *104*, 219-226 und in B. A. Ozenberger, T. J. Brickman, M. A. McIntosh, *J. Bacteriol*. 1989, *171*, 775-783), der ebenfalls eine entA-Negativmutante darstellt. Diese Auswahl ist jedoch nicht limitierend für die vorliegende Erfindung.

Die Herstellung der *trans*-DCHC erfolgt erfindungsgemäß bevorzugt ausgehend von Kohlenhydraten, besonders bevorzugt von Glukose und/oder D-Galaktose und/oder Glyzerin als Kohlenstoffquelle. Dies trifft vor allem für die mikrobielle Herstellung von *trans*-DCHC zu. D-Galaktose und/oder Glyzerin müssen dazu nicht als primäre C-Quelle genutzt werden können, sondern können auch erst während der bereits laufenden Fermentation also während des Wachstums zudosiert werden. Die erfindungsgemäße Herstellung von *trans*-DCHC ausgehend von erneuerbaren C-Quellen wirkt sich insbesondere aufgrund einer enormen Kostenreduzierung gegenüber den herkömmlichen Verfahren, welche die extrem teure Vorstufe Chorismat einsetzen, als besonders vorteilhaft aus.

Das Verfahren zeichnet sich ferner dadurch aus, daß bereits aufgrund einer nur geringfügigen oder bevorzugt gänzlich fehlenden Aktivität der 2,3-Dihydroxybenzoatsynthase (EntA) eine Steigerung des Titers an enantiomerenreinen *trans*-Dihydroxy-Cyclohexadien-Carbonsäuren gegenüber einem nicht genetisch veränderten Organismus wenigstens um den Faktor 10-30 erreicht wird. Hierbei beruht die geringfügige oder ganz fehlende 2,3-Dihydroxybenzoatsynthase-Aktivität auf einer partiellen oder vollständigen, bevorzugt 1 - 99%igen, besonders bevorzugt 5 - 90%igen Inaktivierung des *entA*-Gens, beispielsweise durch Deletion des gesamten Gens oder Teilen davon. Bevorzugt werden sogenannte *entA*-Negativ-Mutanten (*entA*⁻).
In einer bevorzugten Ausführungsvariante der vorliegenden Erfindung wird dieses zuvor genannte Ergebnis an enantiomerenreinen *trans*-Dihydroxy-Cyclohexadien-Carbonsäuren mit einem *E. coli*-Stamm und ausgehend von Glukose als C-Quelle erreicht. Ein besonderer Vorteil des erfindungsgemäßen Verfahrens ist ferner, daß es in den erfindungsgemäßen *E. coli*-Stämmen nicht zu einer Nebenproduktbildung in Form von Chorismat und/oder Isochorismat kommt, d. h. auch in den gewünschten Produkten sind keine Verunreinigungen mit stukturell ähnlichen Verbindungen, wie dem Chorismat und/oder Isochorismat nachweisbar, d.h. sie liegen unterhalb der Nachweisgrenze. Unter der Nachweisgrenze im Sinne der vorliegenden Erfindung ist erfindungsgemäß eine Konzentration an störenden Verbindungen ähnlicher Struktur im Bereich von 0,5 mg/l zu verstehen. D.h. daß Chorismat und/oder Isochorismat als störende Nebenprodukte in Konzentrationen von weniger als 0,5 mg/l Fermentationsmedium vorliegen.

In einer weiteren bevorzugten Ausführungsvariante der vorliegenden Erfindung wird mit einem genetisch veränderten *E. coli*-Stamm, dessen *entA*-Gen inaktiv ist (*entA*^{*-*}) und dessen Gene *entB* und *entC* überexprimiert werden, eine sehr hohe Ausbeute an 2,3-*trans*-DCHC im Bereich mehrerer Gramm pro Liter, nämlich wenigstens 5 g/l erzielt. Hierbei wurde das Kulturmedium nach Pan, J. et al. (Biotechnol. Lett., 1987, 9: 89-94) hinsichtlich seiner Ammoniumkonzentrationen optimiert. Die bevorzugten Stämme weisen gegenüber dem entsprechend nicht genetisch veränderten Organismus erhöhte Aktivitäten der Enzyme Isochorismatsynthase und Isochorismatase und idealerweise keine oder nur eine 2,3-Dihydroxybenzoatsynthase-Aktivität von nahezu Null auf.
Zur näheren Erläuterung der vorliegenden Erfindung sind nachfolgend einige Enzymaktivitäten durch konkrete Meßwerte angegeben. Diese Ausführungen sind beispielhaft und in keiner Weise limitierend für die vorliegende Erfindung. Es handelt sich dabei um Meßwerte, die in dem nicht pathogenen *E. coli*-Stamm AN193 erzielt wurden, der sozusagen der "Kontrollstamm" ist. Dieser Stamm *E. coli* AN193 ist beschrieben bei G. B. Cox et al., *J. Bacteriol.* 1970, *104*, 219-226 und in B. A. Ozenberger, T. J. Brickman, M. A. Mclntosh, *J. Bacteriol*. 1989, *171*, 775-783. Er weist eine spezifische Enzymaktivität der Isochorismatsynthase (chromosomal kodiert durch *entC*) von etwa 0,10 U/mg Protein und der Isochorismatase (chromosomal kodiert durch *entB*) von etwa 0,012 U/mg Protein auf. Durch Überexpression des *entB*-Gens wurde im Vergleich hierzu eine erhöhte Aktivität von etwa 0,29 U/mg Protein und durch Überexpression des *entC*-Gens eine vergleichsweise erhöhte Aktivität von etwa 1,17 U/mg Protein erreicht.

Überraschender Weise führt die Überexpression von *entB* alleine in dem *E. coli*-(*entA*⁻)-Stamm, der eine unveränderte Isochorismatsynthaseaktivität aufweist, nicht zu einer Exkretion von 3,4-*trans*-DCHC. Dies war insbesondere vor dem Hintergrund der bekannten Untersuchungen mit pathogenen *Klebsiella pneumoniae*-Stämmen nicht zu erwarten (Müller, R. et al. Microbiology, 1996, 142: 1005-1012).

Ferner ist überraschend, daß in einer weiteren Ausführungsvariante der vorliegenden Erfindung unter Einsatz eines *E. coli*-Stammes, der idealerweise keine oder nur eine Aktivität der 2,3-Dihydroxybenzoatsynthase (EntA) von nahezu Null (*entA*⁻-Mutante) und eine erhöhte Aktivität der Isochorismatase (EntB) und eine erniedrigte Aktivität der Isochorismatsynthase (EntC) aufweist, die Exkretion von 3,4-*trans*-DCHC in enatiomerenreiner Form erreicht werden kann.

Erfindungsgemäß zeichnet sich das Verfahren vor allem dadurch aus, daß für die chiralen *trans*-Dihydroxy-Cyclohexadien-Carbonsäuren jeweils eine Enatiomerenreinheit (ee) im Bereich von ≥ 99,9 % erreicht wird. Hierbei ist zu betonen, daß erfindungsgemäß keine Verunreinigungen mit Chorismat und/oder Isochorismat als störendem Nebenprodukt ähnlicher Struktur nachweisbar sind, wobei diese Nachweisgrenze bei einem Wert im Bereich von weniger als 0,5 mg/l liegt.

Erfindungsgemäß kann die geringfügige oder fehlende Aktivität der 2,3-Dihydroxybenzoatsynthase (EntA) und/oder veränderte Aktivitäten der Isochorismatase (EntB) bzw. Isochorismatsynthase (EntC) durch Veränderungen auf transkriptionaler, translationaler und/oder posttranslationaler Ebene hervorgerufen werden. Hierunter sind beispielsweise Veränderungen regulativer Elemente, wie z. B. der Promotoraktivitäten, der Beständigkeit der gebildeten mRNAs oder Proteine gegenüber Abbau durch Nukleasen oder Proteasen oder der Inititation der Translation durch Ribosomenbindung oder Verringerung der katalytischen Enzymaktivität selbst oder durch verändertes Bindungsverhalten von Regulatoren oder andere zu verstehen. Darüber hinaus ist auch eine geringfügige oder gänzlich fehlende Aktivität der 2,3-Dihydroxybenzoatsynthase durch die Inaktivierung des *entA*-Gens denkbar, welche erfindungsgemäß bevorzugt ist. Dies kann durch verschiedenartige an sich bekannte gentechnische Ansätze erreicht werden, wie z. B. Insertions- oder Deletionsmutagenese oder chemische Behandlung der Zellen mit mutagenen Agenzien. Bevorzugt sind *entA*-Deletionsmutanten. Die zuvor genannten Methoden dienen dabei lediglich der Erläuterung der Erfindung und wirken nicht limitierend.

Erhöhte Aktivitäten der Enzyme Isochorismatase bzw. Isochorismatsynthase können außerdem durch eine Überexpression der Gene *entB*/*entC* und hier beispielsweise durch eine erhöhte Kopienzahl der Gene, entweder gemeinsam in einem geeigneten Vektor oder separat in verschiedenen geeigneten und untereinander kompatiblen Vektoren, erreicht werden. Denkbar sind aber auch andere Vorgehensweisen, wie z. B. die Expression unter Kontrolle eines starken und/oder induzierbaren Promotors oder die Stabilisierung der gebildeten mRNA und/oder der korrespondierenden Proteine gegenüber Nukleasen und/oder Proteasen. Als Promotoren sind prinzipiell alle Promotoren geeignet, von denen aus eine Expression in dem jeweiligen Wirtsorganismus möglich ist, in den das Genkonstrukt eingebracht wird. Beispielhaft sei hier ein IPTGinduzierbarer Promotor genannt.

Eine erfindungsgemäß verringerte Aktivität der Isochorismatsynthase kann durch eine veränderte Expression des *entC*-Gens, also auf transkriptionaler oder translationaler Ebene, oder eine veränderte Aktivität des bereits gebildeten Enzyms (posttranslational), beispielsweise durch Zusatz spezifischer Effektoren (Inhibitoren) der Isochorismatsynthase erfolgen.
Eine Verminderung der Genexpression, insbesondere der *entC*-Expression, kann Eingriffe auf chromosomaler und/oder plasmidkodierter Ebene erfordern. Hierbei kann die Expression eines Gens durch die Aktivität des Promotors kontrolliert werden, dem das Gen unterliegt. Erfindungsgemäß bevorzugt wird hier ein Promotor, der in seiner Aktivität "leaky" ist, d.h. der derart regulierbar ist, daß die Genexpression stark vermindert ist, idealerweise aber nicht ganz ausgeschaltet wird Unter "leaky" ist somit eine Promotoraktivität zu verstehen, die nicht ganz abgeschaltet ist, sondern noch eine geringe Restaktivität erlaubt und somit "undicht", also etwas durchlässig ist. Unterschiedliche Möglichkeit der Feinregulierung von Promotoren sind dem Fachmann zahlreich bekannt.

Darüber hinaus sind auch der Einsatz und/oder die genetische Veränderung anderer regulatorisch wirkender Elemente, wie u.a. Enhancer oder Silencer, erfindungsgemäß umfaßt.

Ferner ist zur Regulierung der *entC*-Genexpression auch eine Repression der Genexpression durch eine Blockierung der gebildeten mRNA mittels gebildeter antisense-RNA denkbar. Vorgehensweisen, die zu einer sogenannten Antisense-Repression führen, sind dem Fachmann bekannt. Gegenstand der Erfindung sind somit auch die Genkonstrukte und/oder Vektoren zur Erzielung der zuvor beschriebenen veränderten Gen- bzw. Enzymaktivitäten. Im Sinne der vorliegenden Erfindung enthält ein Genkonstrukt wenigstens eine kodierende Nukleotidsequenz der Gene *entB* und/oder *entC* und/oder ggf. *entA* sowie mit diesen operativ verknüpfte regulative Nukleotidsequenzen, wie Promotoren, Enhancer, Silencer, Transkriptionsterminatoren, Targeting-Sequenzen (Transitsignalsequenzen) und/oder Translationsverstärker. Vektoren im Sinne der vorliegenden Erfindung enthalten zusätzliche Nukleotidsequenzen zur Selektion transformierter Wirtszellen und/oder für die Replikation innerhalb der Wirtszelle oder zur Integration in das entsprechende Wirtszell-Genom. Die Herstellung dieser Konstrukte und Vektoren sind dem Fachmann bekannt und werden daher nicht im Detail beschrieben. Hierbei sind wie bereits zuvor erwähnt auch Konstrukte und/oder Vektoren enthaltend Nukleotidsequenzen kodierend für die Isochorismatmutase und/oder Isochorismatsynthase und/oder deren Isoenzyme, z. B. menF, umfaßt.

Ferner ist das erfindungsgemäße Verfahren dadurch gekennzeichnet, daß die gebildeten *trans*-DCHC durch Reaktivextraktion/Reextraktion nahezu quantitativ aus dem Permeat (wässriges Kulturmedium) isoliert werden. Unter Berücksichtigung der Produkteigenschaften in neutraler wässriger Lösung erfolgt diese Isolierung mit Hilfe von kationischen Carriern, d.h. Anionen-selektiven Carriern. Hierbei binden die in wässriger (Kultur)-Lösung als Anionen vorliegenden *trans*-DCHC über ionische Wechselwirkungen an einen kationischen Carrier. Als Carrier werden erfindungsgemäß Ammoniumderivate bevorzugt. Besonders bevorzugt wird Trioctylmethyl-ammoniumchlorid (Aliquat® 336, Aldrich Chemicals Co.). Einer möglicherweise auftretenden Toxizität des Carriers auf das Biosystem, insbesondere bei einer Anwendung in einem integrierten Produktions- und Isolierungsverfahren, wird erfindungsgemäß dadurch begegnet, daß das Konzentrationsgleichgewicht des Carriers in der wässrigen und der organischen Phase derart beeinflußt wird, daß die Carrierkonzentration in der wässrigen Phase des Kulturmediums unter der Toxizitätsgrenze der für das biologische System kritischen Konzentration gehalten wird. Die Vorgehensweisen hierzu sind dem Fachmann geläufig. Erfindungsgemäß bevorzugt werden kationische Carrier und hierbei Ammoniumderivate verwendet, die durch sterisch anspruchsvolle Substituenten nicht oder nur extrem geringfügig membrandurchgängig sind und aufgrund dessen die Toxizitätsgrenze für das biologische System, d.h. den erfindungsgemäß nicht pathogenen Organismus, nicht überschreiten. Unter Membrandurchgängigkeit ist die Einschleusung des Carriers durch die Zellmembran in das biologische System zu verstehen, die passiv durch Diffusion oder aktiv durch Transportvorgänge (z. B. Symport und/oder Antiport) erfolgen kann.
Erfindungsgemäß sind auch komplexere Anionen-selektive Verbindungen als Carrier z. B. aus der Gruppe der Cyclodextrin-Kationen, endrohedralkationische Fullerene, kationische geladene Kronenether und/oder Kryptanden umfaßt.

Erfindungsgemäß werden bei der Reaktivextraktion kationische Carrier in einer Konzentration im Bereich von 1-30 Vol.-%, bevorzugt 3-20 Vol.-%, besonders bevorzugt 5-15 Vol.-% bezogen auf das Kultivierungsmedium eingesetzt. Erfindungsgemäß nimmt dabei die Extraktionsleistung annähernd proportional zur eingesetzten Carrierkonzentration zu. Das erfindungsgemäße Verfahren zeichnet sich ferner dadurch aus, daß bei der Reaktivextraktion als organisches Lösungsmittel Sauerstoff-enthaltende Lösungsmittel mittlerer Kettenzahl mit C₆ bis C₂₀, bevorzugt C₄ bis C₁₂, wie z. B. 2-Undecanon und/oder Diphenylether und/oder Butylbenzol und/oder 1-Octanol eingesetzt werden, wobei 1-Octanol besonders bevorzugt wird.

Als ungeladenes und weitgehend unpolares *trans*-DCHC/Carrierlonenpaar werden beide in eine unpolare, organische Phase überführt. Aus dieser organischen Phase werden die 2,3-*trans*-DCHC dann durch eine dritte Phase (Reinphase) mit hoher Gegenionenkonzentration wieder reextrahiert. Die Triebkraft zur Anreicherung der Produkte in der Reinphase ist das hohe Konzentrationsgefälle der Gegenionen. Es wurden keine Effekte einer irreversiblen Produktbindung an den Carrier beobachtet, so daß die *trans*-DCHC erfindungsgemäß nahezu quantitativ aus der organischen Phase reextrahiert werden können.

Erfindungsgemäß werden bei der Reaktivextraktion als Reextraktionsmittel gesättigte anorganische Salzlösungen, bevorzugt mit Chlorid- und/oder Carbonat-Ionen, besonders bevorzugt in Form einer gesättigten Natriumchloridlösung, eingesetzt. Ferner zeichnet sich das erfindungsgemäße Verfahren dadurch aus, daß die Reextraktionsleistung proportional zur Anionenkonzentration zunimmt und keine direkte Abhängigkeit zum pH-Wert vorliegt.

Ferner hat es sich als vorteilhaft erwiesen die reextrahierten *trans*-DCHC der Reinphase durch Extraktion mit organischen Lösungsmitteln ohne Carrier vom Wasser zu befreien und dann destillativ aufzukonzentrieren. Hierbei treten keine Verluste auf, sofern der Vorgang bei Raumtemperatur und einem sauren pH-Wert durchgeführt wird und keine katalytisch wirkenden Oberflächen, wie z. B. Kieselgele verwendet werden. Das erfindungsgemäße Verfahren ist dabei dadurch gekennzeichnet, daß die Extraktion der enantiomerenreinen *trans*-Dihydroxy-Cyclohexadien-Carbonsäure aus der Salzfracht nahezu quantitativ mit organischen Lösungsmitteln, wie z. B. mittelkettige Alkohole, flüchtige Carbonsäureester, Ketone und/oder funktionalisierte Aromaten, bei einem sauren pH-Wert, bevorzugt im Bereich von pH 0 bis pH 5, besonders bevorzugt bei pH 3 erfolgt. Beispiele für erfindungsgemäß bevorzugte organische Lösungsmittel sind verschiedene Essigester, Aceton, Diphenylether und/oder 1-Butanol, wobei letzteres erfindungsgemäß besonders bevorzugt wird. Diese Angaben dienen der Erläuterung der Erfindung und sind nicht limitierend. Eine schematische Übersicht der erfindungsgemäßen Aufreinigung der *trans*-DCHC ist in Fig. 4 dargestellt.

Besondere Vorteile des erfindungsgemäßen Verfahrens und hier insbesondere bei der Reaktivextraktion/Reextraktion ist, daß die Aufbereitung der *trans*-DCHC unter sehr milden Bedingungen erfolgt, so daß keine Aromatisierung der Produkte eintritt.

In einer weiteren Ausführungsvariante erfolgt die Isolierung der *trans*-DCHC aus dem wässrigen Permeat (Kulturlösung) durch Adsorption/Desorption auf einem Ionenaustauscher, bevorzugt auf einem Anionenaustauscher, wie z. B. auf dem Harz DOWEX 1x8 (100-200 Mesh). Dabei kann die Kulturlösung unmittelbar, d.h. ohne weitere Aufarbeitung auf ein Fließbett oder Festbett aus geeigneten Adsorberpartikeln aufgetragen werden. Gegebenenfalls ist hierzu zunächst eine Abtrennung der partikulären (festen) Bestandteile vorzunehmen, insbesondere bei der Adsorption in einem Festbett. Unabhängig von einer ggf. vorgeschalteten Abtrennung partikulären Bestandteile wird die auf den Adsorber aufzutragende Kulturlösung auf einen pH-Wert im alkalischen Bereich, bevorzugt auf einen pH-Wert im Bereich zwischen 7,5 - 11, besonders bevorzugt von pH 8 eingestellt. Die Leitfähigkeit der Lösung wird durch geeignete Maßnahmen auf einen Wert zwischen 2,5 - 20 mS/cm, bevorzugt 8 - 15 mS/cm, besonders bevorzugt 13,5 mS/cm eingestellt. Ein besonderer Vorteil dieser Ausführungsvariante liegt darin, daß sich die zuvor beschriebene Vorgehensweise erfindungsgemäß durch eine hohe Selektivität auszeichnet und die während der Fermentation gebildeten und in das wässrige Permeat abgegebenen *trans*-DCHC überraschender Weise als überwiegende Komponente auf dem Adsorber gebunden wird. Die Desorption der *trans*-DCHC erfolgt beispielsweise durch Waschen des Adsorbers mit wässrigen Lösungen, welche einen sauren pH-Wert aufweisen. Erfindungsgemäß bevorzugt werden flüchtige Säuren, besonders bevorzugt Ameinsensäure. Zur Isolierung der *trans*-DCHC in fester Form kann beispielsweise eine Gefriertrocknung vorgenommen werden. Überraschender Weise bleiben die *trans*-DCHC durch den Einsatz der zuvor genannten Desorptionsmittel als überwiegend fester Rückstand erhalten. Durch die zuvor beschriebene Vorgehensweise werden erfindungsgemäß Produktreinheiten an *trans*-DCHC von wenigstens 90 % erreicht.

Gegenstand der vorliegenden Erfindung ist auch ein genetisch veränderter Organismus zur verbesserten Herstellung von *trans*-Dihydroxy-Cyclohexadien-Carbonsäuren in enantiomerenreiner Form ohne nachweisbare Verunreinigungen mit Chorismat und/oder Isochorismat, der sich dadurch auszeichnet, daß er ein nicht pathogener Organismus ist, der höchstens den Sicherheitsanforderungen der Risikogruppe 1 zugeordnet ist und im Vergleich zu einem entsprechenden nicht genetisch veränderten nicht pathogenen Organismus keine oder nur eine 2,3-Dihydroxybenzoatsynthase-Aktivität (EntA) von nahezu Null, d.h. idealerweise von Null, und eine gesteigerte Aktivität der Isochorismatase (EntB) und eine gesteigerte oder verminderte Aktivität der Isochorismatsynthase (EntC) aufweist.

Hierbei kann eine nur geringfügige oder fehlende Aktivität der 2,3-Dihydroxybenzoatsyntase dadurch erzielt werden, daß das entsprechende Gen *entA* inaktiviert wurde. Dies kann ungerichtet durch klassische chemische Mutagenese und Selektion auxotropher Mutanten erfolgen oder durch z. B: gerichtete gentechnische Inaktivierungen (z. B. Insertionen, Deletionen) des Gens und anschließende Selektion eindeutig definierter Mutanten. Ferner kann die Expression auf transkriptionaler und/oder tranlationaler Ebene verringert oder blockiert sein. Ebenfalls denkbar ist eine auf Proteinebene (posttranslational) verringerte katalytische Aktivität oder ein verändertes Bindungsverhalten von Effektoren (Aktivatoren oder Inhibitoren).

In einer besonderen Ausführungsvariante der vorliegenden Erfindung zeichnet sich der genetisch veränderte Organismus dadurch aus, daß dessen *entA*-Gen kodierend für die 2,3-Dihydroxybenzoatsyntase deletiert ist und dessen Gene *entC* und *entB* kodierend für die Isochorismatsynthase und Isochorismatase verstärkt exprimiert werden.
Erfindungsgemäß ist dabei ein genetisch veränderter Organismus umfaßt, bei dem das *entA*-Gen vollständig oder partiell, bevorzugt über einem Bereich von wenigstens 1-99% und bevorzugt 5-90% deletiert ist und das *entB*-Gen verstärkt exprimiert wird und die Expression des *entC*-Gens, je nach gewünschtem Produkt, verstärkt oder vermindert wird. Ferner kann die Aktivität der Isochorismatsynthase kodiert durch *entC* auch auf translationaler und/oder posttranslationaler Ebene reguliert werden. Hierbei sind Vorgehensweisen zur Antisense-Repression oder eine Hemmung mit spezifischen Effektoren denkbar.

In einer weiteren Variante der vorliegenden Erfindung ist ein genetisch veränderter Organismus umfaßt, enthaltend wenigstens eines der Gene *entB* und/oder *entC* und/oder Homologe davon, wobei die Gene sowohl aus homologen als auch heterologen Organismen isoliert sein können.
Unter homologen Nukleotidsequenzen sind erfindungsgemäß solche Sequenzen zu verstehen, die zu den Nukleotidsequenzen des verwendeten Wirtsorganismus komplementär sind und/oder mit diesen Nukleotidsequenzen hybridisieren. Der Begriff hybridisierende Sequenzen schließt erfindungsgemäß substanziell ähnliche Nukleotidsequenzen aus der Gruppe von DNA oder RNA ein, die unter an sich bekannten stringenten Bedingungen eine spezifische Wechselwirkung (Bindung) mit den zuvor genannten Nukleotidsequenzen eingehen. Die in den erfindungsgemäßen Organismus eingebrachten Nukleotidsequenzen kodierend für die Gene *entB* oder *entC* können natürlich vorkommende Sequenzen sowie künstliche, z. B. durch chemische Synthese erhaltene und gegebenenfalls an den Kodon-Gebrauch des Wirtsorganismus angepaßte Nukleotid-Sequenzen sein. Unter homologen Organismen sind solche zu verstehen, die einer verwandten Familie angehören. Entsprechend repräsentieren heterologe Organismen weniger verwandte Vertreter.

Erfindungsgemäß zeichnet sich ein genetisch veränderter Organismus der zuvor beschriebenen Art dadurch aus, daß er durch eine verstärkte Expression des *entC*-Gens, zusammen mit der verstärkten Expression des *entB*-Gens in einer *entA*-Negativ-Mutante, vermehrt 2,3-*trans*-Dihydroxy-Cyclohexadien-Carbonsäure bildet.

In einer weiteren Varianten eines erfindungsgemäß genetisch veränderten Organismus zeichnet sich dieser dadurch aus, daß er aufgrund einer verminderten Expression des *entC*-Gens (bei gleichzeitiger verstärkter *entB*-Expression in einer *entA*-Negativ-Mutante) vermehrt 3,4-*trans*-Dihydroxy-Cyclohexadien-Carbonsäure bildet. Hierbei kann die verminderte Expression des *entC*-Gens beispielsweise durch eine regulierbare Promotoraktivität erreicht werden, der das *entC*-Gen unterliegt. Diese Promotoraktivität kann "leaky" sein, d.h. sie kann sehr gering sein, muß aber nicht zwingendermaßen zu einer 100%igen Blockade der Expression führen. Ferner ist eine Antisense-Represson des *entC*-Gens denkbar. Prinzipiell wird hierbei anhand der kodierenden *entC*-Gensequenz eine mRNA transkribiert, die dann mit einer entsprechenden antisense-mRNA hybridisiert und aufgrund dessen nicht translatiert werden kann, so daß das *entC*-Gen nicht zur Ausprägung kommt. Die entsprechende antisense-mRNA kann beispielsweise anhand eines Genkonstrukts enthaltend das *entC*-Gen in "antisense"-Orientierung unter Kontrolle eines, bevorzugt regulierbaren, Promotors gebildet werden, wobei dieses Genkonstrukt nach allgemein bekannten Methoden der Gentechnik hergestellt und in den erfindungsgemäß nicht pathogenen genetisch veränderten Organismus übertragen wird.
Generell sind alle an sich bekannten regulativen Elemente erfindungsgemäß erfaßt, die die Expression von Genen in den Wirtszellen kontrollieren können. Hierzu zählen neben Promotoren u.a. auch Enhancer oder Silencer.

Generell kann eine verringerte Isochorismatsynthase-Aktivität auch durch eine Regulation auf posttranslationaler Ebene zu einer erfindungsgemäß vermehrten Bildung von 3,4-*trans*-DCHC führen, beispielsweise durch die Zugabe von spezifische Effektoren zum Kulturansatz.

Gegenstand der vorliegenden Erfindung ist ferner ein genetisch veränderter Organismus, der ein Genkonstrukt enthält, das wenigstens einen regulierbaren Promotor aufweist, der am 5'-Ende mit dem *entC*-Strukturgen operativ verknüpft ist. Hierbei liegt das *entC*-Strukturgen in der sogenannten "sense"-Orientierung vor. Erfindungsgemäß umfaßt ist auch ein genetisch veränderter Organismus, enthaltend ein Genkonstrukt, welches selbst einen, bevorzugt regulierbaren, Promotor enthält, der operativ mit einer *entC*-Nukleotidsequenz in "antisense"-Orientierung verknüpft ist.

Unter einer operativen Verknüpfung versteht man die sequenzielle Anordnung beispielsweise von Promotor, kodierender Sequenz, Terminator und ggf. weiterer regulatorischer Elemente derart, daß jedes der regulatorischen Elemente seine Funktion bei der Expression der kodierenden Sequenz bestimmungsgemäß erfüllen kann. Diese regulatorischen Nukleotidsequenzen können natürlichen Ursprungs sein oder durch chemische Synthese erhalten werden. Als Promotor ist grundsätzlich jeder Promotor geeignet, der die Genexpression in dem entsprechenden Wirtsorganismus steuern kann. Hierbei kann es sich erfindungsgemäß auch um einen chemisch induzierbaren Promotor handeln, durch den die Expression der ihm unterliegenden Gene in der Wirtszelle zu einem bestimmten Zeitpunkt gesteuert werden kann.
Beispielhaft sei hier ein durch IPTG (Isopropyl-β-thiogalactosid) induzierbarer Promotor genannt.
Die Herstellung einer Genstruktur erfolgt durch Fusion eines geeigneten Promotors mit wenigstens einer Nukleotidsequenz nach gängigen Rekombinations- und Klonierungstechniken, wie sie beispielsweise in Sambrook, J. et al., Molecular Cloning: A Laboratory Manual, Cold Spring Harbor Laboratury, Cold Spring Harbor, NY (1989) beschrieben sind. Für die Verbindung der DNA-Fragmente miteinander können an die Fragmente Adaptoren oder Linker angesetzt werden.

Darüber hinaus betrifft die vorliegende Erfindung einen Vektor enthaltend wenigstens eine Nukleotidsequenz der zuvor beschriebenen Art, mit diesen operativ verknüpfte regulative Nukleotidsequenzen sowie zusätzliche Nukleotidsequenzen zur Selektion transformierter Wirtszellen, für die Replikation innerhalb der Wirtszelle oder zur Integration in das entsprechende Wirtszell-Genom. Ferner kann der erfindungsgemäße Vektor eine Genstruktur der vorgenannten Art enthalten.

Die genetischen Veränderungen des erfindungsgemäßen Organismus können chromosomal und/oder extrachromosomal, und hierbei bevorzugt plasmidkodiert, vorliegen.

Gegenstand der Erfindung sind ferner enantiomerenreine *trans*-Dihydroxy-Cyclohexadien-Carbonsäuren hergestellt durch ein Verfahren der zuvor genannten Art, die eine Säurekonstante pKₛ im Bereich zwischen 3 und 5 aufweisen und bei Raumtemperatur und einem pH-Wert im Bereich von 4 bis 13, bevorzugt 5 bis 11, mit Zerfallkonstanten von weniger als 0,1 d⁻¹ entsprechend einer Halbwertszeit von wenigstens 7 Tagen sehr stabil sind.

Erfindungsgemäß umfaßt ist außerdem die Verwendung der jeweiligen enantiomerenreinen *trans*-Dihydroxy-Cyclohexadien-Carbonsäure zur Herstellung von komplexen biologisch aktiven Stoffwechselmetaboliten, z. B. Pflanzenmetaboliten, besonders bevorzugt Cyclohexandiol-Epoxiden und/oder von Kohlenhydratmimetika, höchst bevorzugt Carbazucker und insbesondere Amino-Carbazucker.

Die Verwendung von *cis*-Dihydroxy-Cyclohexadien-Carbonsäuren ist ebenfalls bekannt. Allerdings beruht die Darstellung der *cis*-DCHC auf einer oxidativen Dioxygenierung von Aromaten, welche in einem fermentativen Herstellungsprozeß dem Medium zugefügt werden müssen. Durch die erfindungsgemäß fermentative Bereitstellung enantiomerenreiner *trans*-DCHC in hohen Ausbeuten eröffnen sich nun wesentlich wirtschaftlichere Möglichkeiten und z. T. völlig neue Wege der Natur- und Wirkstoffsynthese.

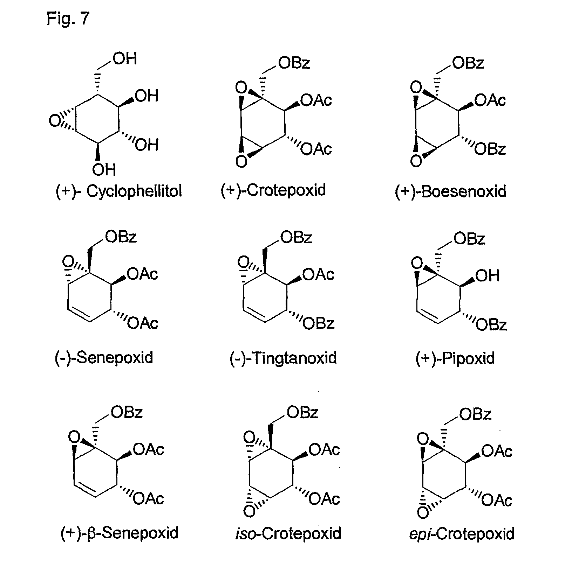

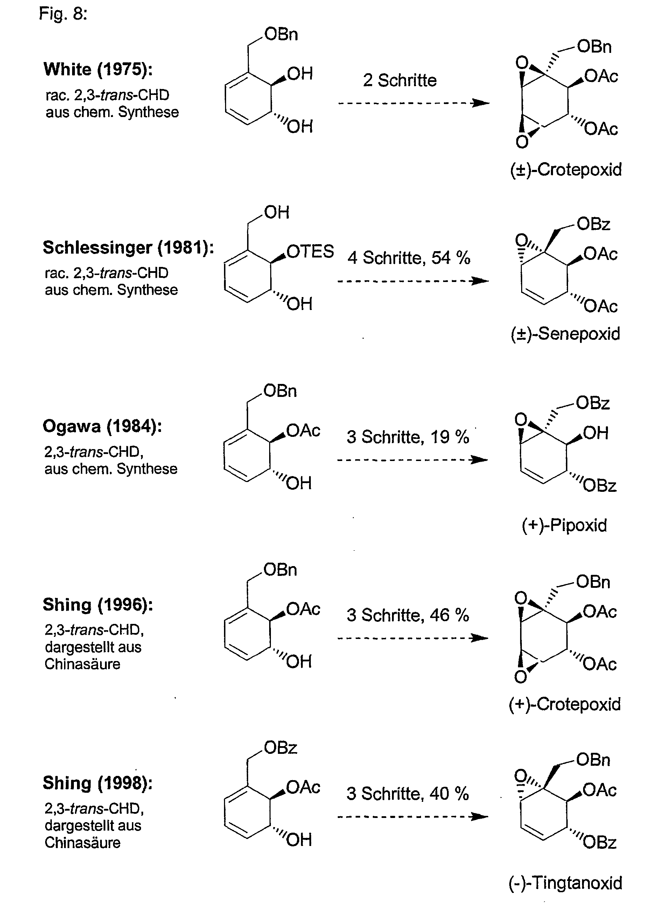

So können die erfindungsgemäßen *trans*-DCHC zum Beispiel als Ausgangsverbindungen zur Darstellung von Derivaten biologisch aktiver Stoffwechselmetabolite, z. B. Pflanzenmetabolite, verwendet werden. Charakteristisch für die Stoffklasse dieser Metabolite ist das 2,3-Dihydroxycyclohexylmethanol-Grundgerüst, welches an 4-, 5- oder 6-Position epoxidiert ist. Bekannteste Vertreter sind u. a. Crotepoxid, iso-Crotepoxid, epi-Crotepoxid, Senepoxid, β- Senepoxid, Pipoxid, Cyclophellitol, Boesenoxid und Tingtanoxid, die in Fig. 7 dargestellt sind .
Für diese Stoffklassen konnte gezeigt werden, daß sie als Kohlenhydratmimetika inhibitorische Wirkung, z. B. Tumor-inhibitorische Wirkung auf verschiedenartige Karzinome, zeigen und somit für die pharmazeutische Anwendung potentiell geeignet sind. Die Darstellung erfolgt bislang durch aufwendige chemische Synthesen mit den bekannten Nachteilen, wie hoher Kosten und Zeitaufwand, geringe Enantioselektivität, um nur die wichtigsten zu nennen. Bei diesen Synthesen treten als Zwischenverbindungen auch Derivate der *trans*-DCHC auf. Eine Übersicht über wichtige Synthesen der Cyclohexandiol-Epoxid-Pflanzenmetabolite, bei denen ein 2,3-*trans*-DCHC-Derivat jeweils eine Schlüsselsubstanz darstellt ist in Fig. 8 dargestellt. Die erfindungsgemäße Bereitstellung der *trans*-DCHC ermöglicht somit ein weites Einsatzspektrum dieser Verbindungen und trägt zu wesentlichen Vereinfachungen der aufwendigen Synthesen komplexer Natur- und Wirkstoffe bei, da die Synthesen nun direkt von den fermentativ hergestellten erfindungsgemäßen *trans*-DCHC ausgehen können und vorausgehende aufwendige Synthese- und Aufreinigungsschritte zur Bereitstellung der *trans*-DCHC aufgrund der vorliegenden Erfindung nicht mehr erforderlich sind und entfallen. Die vorliegende Erfindung birgt somit ein enormes wirtschaftliches Potential in sich.

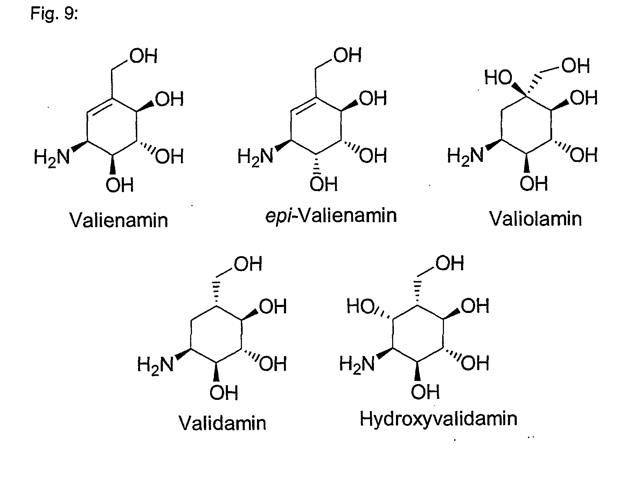

Darüber hinaus ist ausgehend von den erfindungsgemäßen *trans*-DCHC auch die Synthese von Carbazuckern und insbesondere Amino-Carbazuckern möglich, die ebenfalls als Kohlenhydratmimetika ein hohes pharmazeutisches Potential besitzen. Wichtige Vertreter dieser Stoffklasse sind z. B. Valienamine (Fig. 9).
Valienamin-Derivate sind insbesondere als Ausgangsstoffe zur Synthese von Kohlenhydrat-Mimetika, wie z.B. Acarbose oder Voglibose, welche u.a. als α-Glucosidaseinhibitor zur Bekämpfung von Altersdiabetes oder Fettsüchtigkeit Einsatz finden, höchst interessant.

Gegenstand der vorliegenden Erfindung ist auch die Verwendung der *trans*-DCHC (z. B. gebunden an einen polymeren Träger) zur Identifizierung und/oder Darstellung weiterer Wirkstoffe, z. B. Polysaccharid-Mimetika und/oder zum Aufbau von Substanzbibliotheken, z. B. für sogenannte "high throughput screening"-Experimente.

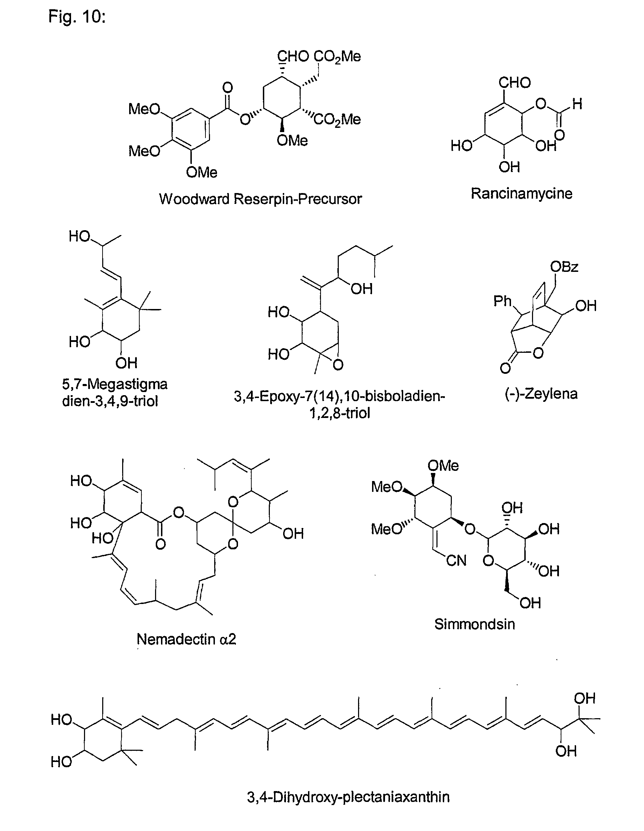

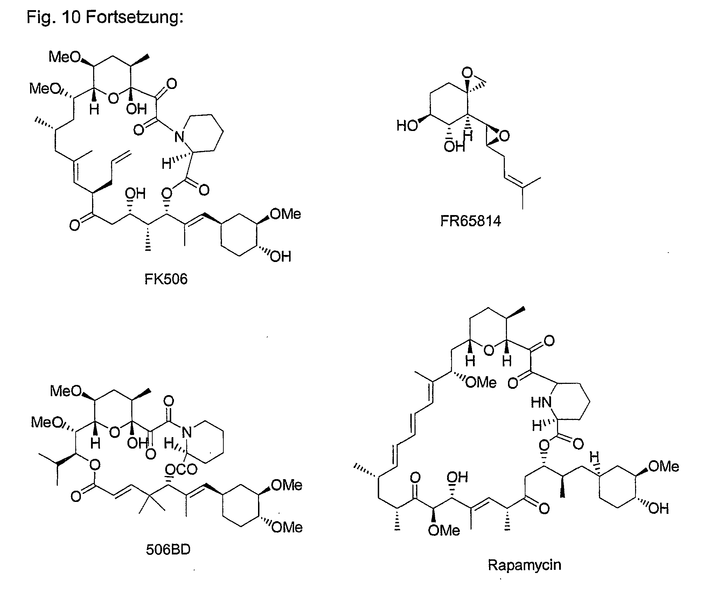

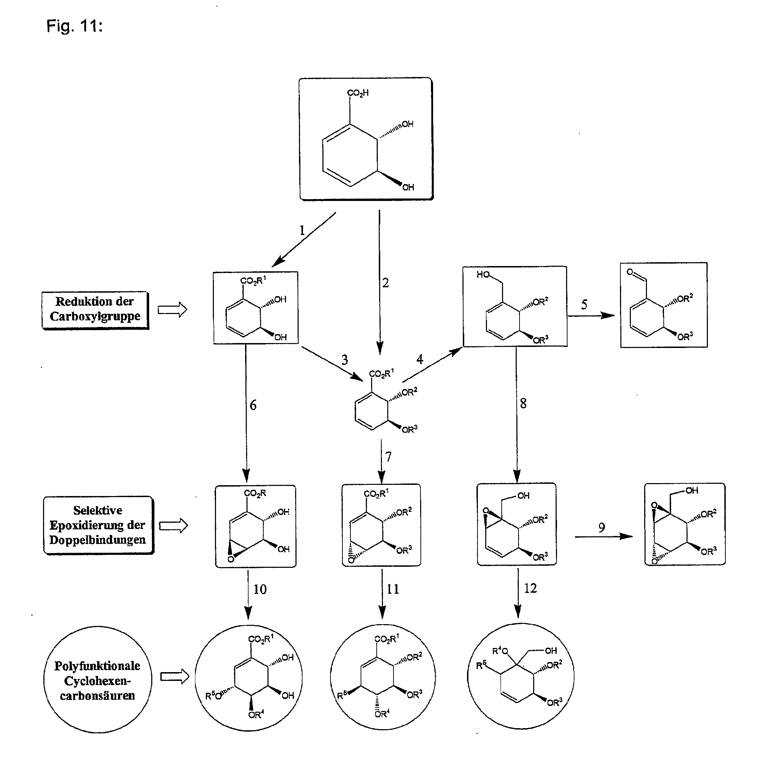

Als weitere Verwendungsmöglichkeiten der erfindungsgemäßen *trans*-DCHC ist ein großes Spektrum an biologisch aktiven Substanzen denkbar, wie z. B. die Wirkstoffklasse der Immunosuppressiva oder Antitumor-Pharmaka um nur einige zu nennen. Eine Auswahl solcher Verbindungen ist in Fig. 10 dargestellt.
Darüber hinaus ist ausgehend von den erfindungsgemäßen *trans*-DCHC eine weitere Vielzahl an komplexen Verbindungen darstellbar, die hier allgemein als Folgechemie bezeichnet wird. Hierbei ist erfindungsgemäß insbesondere eine gezielte Reaktionsführung, z.B. durch selektive Reduzierung der Carboxylgruppe, selektive Epoxidierung der Doppelbindungen und/oder weitere Folgemodifikationen möglich. Ein Ausblick denkbarer folgechemischer Verbindungen sind am Beispiel der erfindungsgemäßen 2,3-*trans*-DCHC als Ausgangssubstanz in Fig. 11 zusammengefaßt.

Die nachfolgenden Ausführungsbeipiele sollen die vorliegende Erfindung näher erläutern, ohne jedoch limitiernd zu wirken.

### 1. Allgemeine genetische Techniken:

Die Isolierung von genomischer DNA und Plasmid-DNA sowie alle Techniken zur Restriktion, Klenow- und alkalische Phosphatasebehandlung, ferner Methoden zur Klonierung und Sequenzierung und Amplifikation (PCR) von DNA sowie die Transformation, Kultivierung und Selektion von Wirtszellen wurden nach Sambrook, J. et al. (Molecular Cloning: A Laboratory Manual, Cold Spring Harbor Laboratory, Cold Spring Harbor, NY, 1998) durchgeführt.

### 2. Isolierung der Gene entB und entC und Klonierung in den Expressionsvektor pJF119EH1:

Die Isolierung der Gene *entB* und *entC* erfolgte aus *E. coli* Stamm W3110 (J. Lederberg, Yale University, USA). Eine Beschreibung dieses Wildstammes findet sich bei C. W. Hill, B. W. Harnish, *Proc. Natl. Acad. Sci. USA* 1981, 78, 7069-7072. Die Präparation der chromosomalen DNA erfolgte gemäß einem Standardprotokoll aus R. Süßmund, J. Eberspächer, R. Haag, W. Springer, *Mikrobiologisch-Biochemisches Praktikum,* 2. Auflage, Thieme Verlag, Stuttgart, New York, 1999. Von der chromosomalen DNA wurde *entB* durch PCR amplifiziert. Die verwendeten Oligonucleotide waren TAT GGA TCC ACG CGC ATC AGC CTG AA und GGG CTG CAG ACA TTT TTA CCG CTG. Die PCR-Bedingungen waren: Denaturieren 5 min (94 °C), 34 Zyklen: - Denaturieren 4 min (94 °C), Annealen 2 min (57 °C), Prolongieren 1.5 min (75 °C) -, Halten 7 min (75 °C), Kühlen auf 4 °C. Das amplifizierte *entB*-Fragment (855 bp) wurde durch Gelelektrophorese (0.8 % Agarose-Gel, Ethidiumbromid-Detektion) isoliert und durch Affinitätschromatographie gereinigt (Qiagen Extraction Kid, Qiagen). Das Fragment wurde mit den Endonucleasen *Bam*HI und *Pst*I geschnitten und gereinigt durch Ionentauscherchromatographie (midi-Kid, Qiagen).
Das Gen *entC* wurde auf entsprechende Weise generiert. Mit chromosomaler DNA aus *E. coli* W3110 wurde eine PCR mit den Oligonucleotiden GGC GAG CTC ATT ATT AAA GCC TTT und TGC GGA TCC TCG CTC CTT ATT GC durchgeführt. Die PCR-Bedingungen waren: 2 min (94 °C), 10 Zyklen: - Denaturieren 0.5 min (94 °C), Annealen 0.75 min (40 °C), Prolongieren 1 min (72 °C) -, 20 Zyklen: - Denaturieren 0.5 min (94 °C), Annealen 0.75 min (55 °C), Prolongieren 1 min + 10 sec/Zyklus (72 °C)-, Kühlen auf 4 °C.
Das DNA-Produkt (1220 bp) wurde wie *entB* elektrophoretisch gereinigt und extrahiert. Das Produkt wurde mit den Endonucleasen *Bam*HI und *Sac*I geschnitten und durch lonentauscherchromatographie gereinigt.

### Konstruktion der Plasmide pDF1 und pDF2

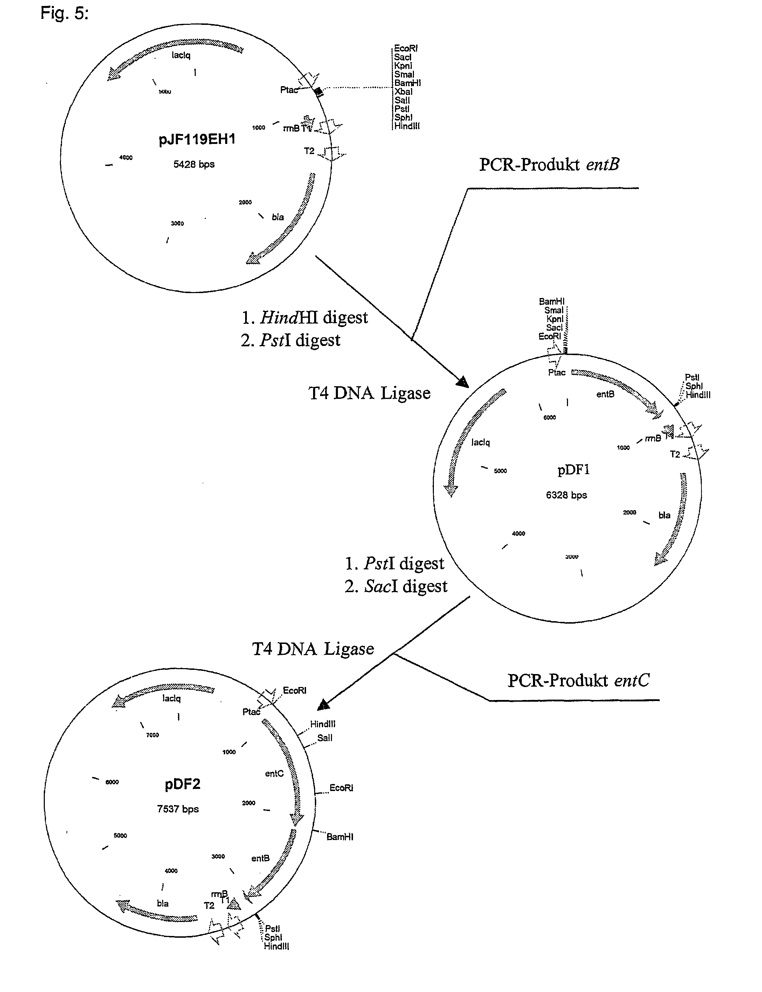

Das *entB* Gen wurde mit T4 DNA-Ligase in den geschnittenen Vektor pJF119EH1 (Fürste, J. P. et al., Gene, 1986, 48: 119-131) ligiert (Schnittstellen *Bam*HI, *Pst*I). Das Plasmid wurde in den Klonier- und Expressionsstamm *E. coli* DH5α transformiert und anschließend auf AgarPlatten (LB, 100 mg/l Ampicillin) zu Einzelkolonien kultiviert. Das Plasmid pDF1 (pJF119EH1 mit Insert *entB*) wurde selektiert und durch eine Restriktionsanalyse verifiziert.
In analoger Weise wurde das *entC*-Gen in den geschnittenen Vektor pDF1 (Schnittstellen *Pst*I, *Sac*I) insertiert. Verifikation des DNA-Produktes erfolgte wieder durch Restriktionsanalyse. Mit beiden Plasmiden wurden entlang der insertierten Gene eine Sequenzierung durchgeführt. Eine Illustration der Plasmidkonstruktion ist in Fig. 5 dargestellt.
Die Plasmide wurden in die *E. coli*-Stämme AN193 und H1882 transformiert. Die Expression von aktiven Proteinen wurde durch SDS-PAGE und Enzymaktivitätstests bestätigt. Die verwendeten *E. coli*-Stämme sind wie folgt charakterisiert:
Der Stamm AN193 entspricht Elter AB1515, hat jedoch eine Anzahl an Deletionen (*trp leu proC lac fhuA rpsL entA403)*. Der Stamm AN193 ist beschrieben bei G. B. Cox et al., *J. Bacteriol*. 1970, *104*, 219-226 und in B. A. Ozenberger, T. J. Brickman, M. A. Mclntosh, *J. Bacteriol*. 1989, *171*, 775-783. Der Stamm H1882 entspricht dem Stamm MC4100 (DSM 6574, ATCC 35695), hat aber die Dihydroxybenzoatsynthase-Defizienz (*Δ*(*fepA-ent*)). Nähere Beschreibung zu dem Stamm MC4100 findet sich bei M. J. Casadaban, *J. Mol. Biol.* 1976, *104*, 541-555 und bei M. J. Casadaban, S. N. Cohen, *Proc. Natl. Acad. Sci USA* 1979, *76,* 4530-4533, sowie bei Y. Komeda, T. lino, *J. Bacteriol* 1979, *139*, 721-729.

### 3. Fermentation zur Herstellung von trans-DCHC:

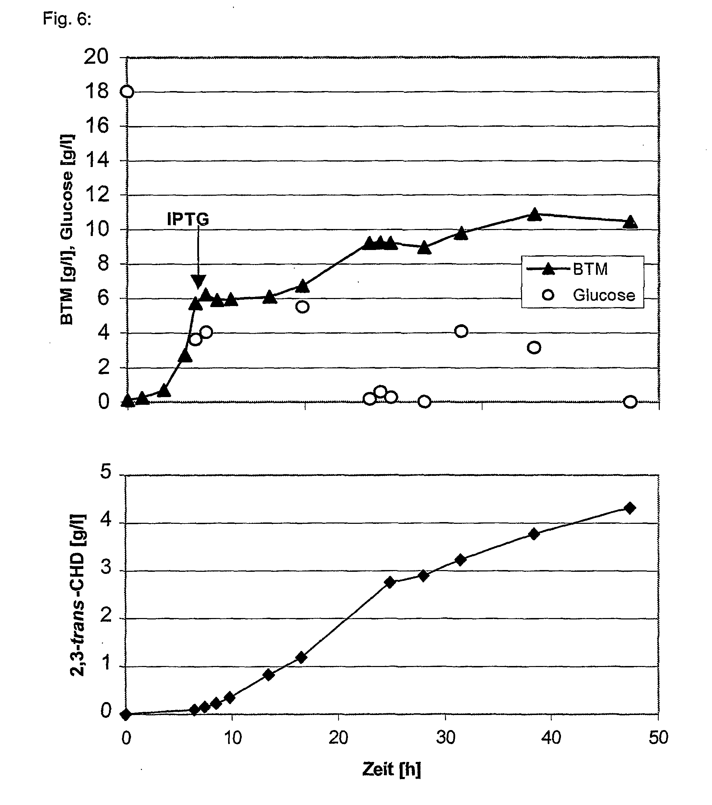

100 µl einer 50 %-Glycerin-Kultur (*feed-stock*) des Stammes AN193/pDF2 dient zur Inokulation von Vorkultur 1 bestehend aus 5 ml Hefeextraktmedium (Hefeextrakt 5 g/l, Caseinpepton 10 g/l, NaCl 5 g/l, Ampicillin 100 mg/l) in einem 20 ml Reagenzglas. Die Zellen wurden kultiviert bei 37 °C unter Schütteln (150 rpm) für 12 h. Mit 500 µl von Vorkultur 1 wurde das Medium für Vorkultur 2 inokuliert, bestehend aus 3 x 100 ml Hefeextraktmedium (Hefeextrakt 5 g/l, Caseinpepton 10 g/l, NaCl 5 g/l, Ampicillin 100 mg/l) in 1 Liter schikanierten Schüttelkolben. Kultivierung der Zellen erfolgte bei 37 °C unter Schütteln (150 rpm) für 12 h. Vorkultur 2 wurde überführt in einen 7.5 Liter belüfteten und pHkontrollierten Rührkesselreaktor mit 5 Liter steril-filtriertem Hauptkulturmedium (KH₂PO₄ 13 g/l, K₂HPO₄ 10 g/l, NaH₂PO₄·2H₂O 6 g/l, (NH₄)₂PO₄ 2 g/l, NH₄Cl 0.2 g/l, MgSO₄·7H₂O 3 g/l, Spurenlösung 1 ml/l, Ampicillin 100 mg/l, Thiamin 20 mg/l, Leucin 20 mg/l, Prolin 20 mg/l, Adenin 20 mg/l, Tryptophan 20 mg/l, 2,3-Dihydroxybenzoesäure 20 mg/l, Glucose 30 g/l).
Die Spurenlösung enthielt in 5 N HCl-Lösung gelöst: FeSO₄·7H₂O 40g/l, CaCl₂H₂O 40 g/l, MnSO₄nH₂O 10 g/l, AlCl₃6H₂O 10 g/l, CoCl₂ 4 g/l, ZnSO₄·7H₂O 2 g/l, Na₂MoO₄H₂O 2 g/l CuCl₂·2H₂O 1 g/l, H₃BO₃ 0.5 g/l.
Die Fermentation wurde mit einem Gasstrom von 4.03 l/min belüftet. Der pH wurde mit 25 % wäßriger Ammoniaklösung auf 7.0 geregelt. Die Temperatur wurde auf 37 °C eingestellt. Die Rührgeschwindigkeit betrug 500 rpm. Die Konzentration an gelöstem Sauerstoff wurde durch Regulation der Zuluft und der Rührdrehzahl auf größer 20 % Luftsättigung eingestellt. Antischaum AF 298 (Sigma) wurde bei Bedarf zugegeben. Nach Erreichen einer Biotrockenmassen von 6 g/l wurde IPTG (132 mg in 5 ml Wasser) zur Induktion steril-filtriert zugegeben. Nach Verbrauch der Anfangsglucose wird ein sterilisiertes Glucosekonzentrat (700 g/l) zugefüttert. Der Flow betrug 0.17 ml/min. Die Konzentration an 2,3-*trans*-DCHC betrug nach 50 h Prozeßzeit 930 mg/l (6 mM).
Durch weitere Optimierung der Medienzusammensetzung, beispielsweise durch Erhöhung der Ammoniumkonzentration und Optimierung der Fermentationsbedingungen, insbesondere des pH-Wertes und der Temperatur, kann eine Erhöhung der Biotrockenmasse (über 20 g/l BTM) erzielt werden. Mit höheren Biotrockenmassen können höhere Produktbildungsraten (größer 60 mgl⁻¹h⁻¹g(BTM)⁻¹), einhergehend mit höheren Produkttiter (über 5 g/l für 2,3-*trans*-DCHC mit Stamm AN193/pDF2) erreicht werden. Eine Erhöhung der Biotrockenmassen kann insbesondere auch dadurch erreicht werden, daß nach oder während der Wachstumsphase statt Glucose andere Kohlenstoffquellen zur Produktion von *trans*-DCHC eingesetzt werden, insbesondere D-Galactose oder Glycerin.
Die Prozeßdaten einer beispielhaften Fermentation unter weiter optimierten Bedingungen ist in Fig. 6 zu sehen.

### 4. Reaktivextraktion der 2,3-trans-DCHC und Abtrennung der Salzfracht:

Der kationische Carrier Aliquat® 336 wurde zu 5 Vol.% in dem organischen Lösungsmittel 1-Octanol bei pH 7 gelöst und zur Extraktion der gebildeten 2,3-*trans*-DCHC aus dem Kulturmedium (Fermentationspermeat) eingesetzt.
Die Reextraktion (Rück-Extraktion) von 2,3-*trans*-DCHC erfolgte mit gesättigter Natriumchloridlösung bei pH 7.

Die Reinphase der Reaktivextraktion enthält neben den *trans*-DCHC größere Mengen Salze in wäßriger Lösung. Durch Extraktion mit organischen Lösungsmitteln läßt sich das Produkt quantitativ von der Salzfracht trennen. Dies erfolgte mit 1-Butanol bei pH 3, wobei Extraktionskoeffizienten von wenigstens 30 % erreicht wurden. Nach dem Trocknen des Produktes mit Magnesiumsulfat über Nacht, anschließende Filtration und Trocknung im Vakuum wird das Produkt salz- und wasserfrei erhalten. Im 1H-NMR- und 13C-NMR-Spektrum des so erhaltenen weißgelben Feststoffes sind keine Verunreinigungen, insbesondere durch Chorismat und/oder Isochorismat, festzustellen.

### 5. Isolierung der 2,3-trans-DCHC durch Adsorption und Desorption:

2,2 I der Kulturlösung werden nach der Fermentation durch Zentrifugation von festen partikulären Bestandteilen befreit und auf einen pH-Wert von 8 und eine Leitfähigkeit von 13,5 mS/cm eingestellt. Als Adsorber werden 530 ml DOWEX 1x8 (CL-Form, 100-200 Mesh) in eine Säule zu 27 cm sedimentierter Bettlänge gefüllt und mit 50 mM Di-Kaliumhydrogenphosphatpuffer (pH 8) equilibriert. Der Adsorber wird mit 2,2 I der Kulturlösung bei 2,4 x 10⁻⁴ m/s beladen. Nach erfolgter Beladung wird mit dem 5-fachem Säulenvolumen an 50 mM Di-Kaliumhydrogenphosphatpuffer (pH 8) bei gleicher Fließgeschwindigkeit gewaschen. Anschließend erfolgt die Desorption bei gleicher Fließgeschwindigkeit mit dem 8-fachen Säulenvolumen 50 mM Ameisensäure. Die erhaltene Desoptionsfraktion wird gefriergetrocknet. Bei einer Analyse durch ¹H-NMR und HPLC-Chromatographie des erhaltenen Feststoffes sind keine Verunreinigungen festzustellen.

### Legende zu den Figuren:

- Fig. 1:: Strukturformeln der *trans*-Dihydroxy-Cyclohexadien-Carbonsäure-Stereoisomere. Dargestellt sind (5*S*,6*S*)-Dihydroxy-cyclohexa-1,3-diencarbonsäure und (3*R*,4*R*)-Dihydroxy-cyclohexy-1,5-diencarbonsäure.
- Fig. 2:: Übersicht über den Shikimatbiosyntheseweg. Chorismat ist Schlüsselsubstanz der Biosynthese der aromatischen Aminosäuren, des Chelatbildners Enterobactin sowie der Folate, der Menaquinone und der Ubiquinone.
- Fig. 3:: Biosyntheseweg von Chorismat zu 2,3-*trans*-DCHC (über Isochorismat) und 3,4-*trans*-DCHC, *entC* kodiert Isochorismatsynthase, *entB* kodiert Isochorismatase, *entA* kodiert 2,3-Dihydroxybenzoatsynthase.
- Fig. 4:: Aufreinigung von *trans*-DCHC durch Reaktivextraktion. Die organische Phase dient als sogenannte "selektive Drehtür" zur Produktextraktion. *trans*-DCHC werden aus der wässerigen Phase als lipophiles lonenpaar Carrier-DCHC durch die organische Phase in die wäßrige Produktphase transportiert. Triebkraft der Anreicherung der *trans*-DCHC in der Produktphase ist das Konzentrationsgefälle an Gegenionen. Der Ladungsausgleich beider Phasen erfolgt durch Rücktransport des Chlorids.
- Fig. 5:: Schematische Darstellung der Konstruktion der Plasmide pDF1 und pDF2.
- Fig. 6:: Beispiel einer Fermentation von *E. coli* Stamm AN193 mit Plasmid pDF2 zur Produktion von 2,3-*trans*-DCHC. Dargestellt ist der zeitliche Verlauf der Biotrockenmasse, der Glucosekonzentration und des Produkttiters.
- Fig. 7:: Strukturformeln biologisch aktiver Pflanzenmetabolite. Die Grundstruktur des 2,3-Dihydroxy-cyclohexylmethanol-Gerüst mit *trans*-ständiger, vicinaler Diol-Einheit ist in allen Stubstanzen enthalten.
- Fig. 8:: Übersicht über wichtige Synthesen der Cyclohexandiol-Epoxid-Pflanzenmetabolite. Schlüsselsubstanz ist jeweils ein Derivat der 2,3-*trans*-DCHC.
- Fig. 9:: Übersicht von Strukturformeln der Stoffklasse der Amino-Carbazucker.
- Fig. 10:: Übersicht von Strukturformeln weiterer Zielklassen.
- Fig. 11:: Übersicht von allgemeinen Strukturformeln von Ziel-Verbindungen (Folgechemie), die ausgehend von den erfindungsgemäßen *trans-* DCHC gezielt, d.h. durch selektive Reduktion, Epoxidierung und Modifikation hergestellt werden können. Beispielhaft ist als Ausgangssubstanz eine 2,3-*trans*-Dihydroxy-Cyclohexadien-Carbonsäure dargestellt.

## Patentansprüche

1. Verfahren zur verbesserten Herstellung von *trans*-Dihydroxy-Cyclohexadien-Carbonsäuren und/oder deren Folgeprodukte, **dadurch gekennzeichnet, daß** die Synthese der *trans*-Dihydroxy-Cyclohexadien-Carbonsäuren in enantiomerenreiner Form ohne nachweisbare Verunreinigungen mit Chorismat ausgehend von erneuerbaren Kohlenstoffquellen durch Kultivierung wenigstens eines genetisch veränderten Organismus der Gattung der Enterobakterien erfolgt, welcher höchstens den Sicherheitsanforderungen der Risikogruppe 1 zugeordnet wird, welcher über einen Aromatenstoffwechsel verfügt und im Vergleich zu einem entsprechend nicht genetisch veränderten Organismus
a) eine erhöhte Aktivität der Isochorismatsynthase (EntC) und Isochorismatase (EntB) im Vergleich zu *E. coli*-Stamm AN193, hervorgerufen durch Veränderungen auf transkriptionaler Ebene, und keine Aktivität der 2,3-Dihydroxybenzoatsynthase (EntA) durch eine vollständige Inaktivierung des Gens kodierend für die 2,3-Dihydroxybenzoatsynthase (*entA*) aufweist oder
b) eine erhöhte Aktivität der Isochorismatase (EntB), hervorgerufen durch Veränderungen auf transkriptionaler Ebene und eine erniedrigte Aktivität der Isochorismatsynthase (EntC), hervorgerufen durch Veränderungen auf transkriptionaler Ebene oder translationaler Ebene mittels antisense-RNA im Vergleich zu *E. coli*-Stamm AN193, und keine Aktivität der 2,3-Dihydroxybenzoatsynthase (EntA) durch eine vollständige Inaktivierung des Gens kodierend für die 2,3-Dihydroxybenzoatsynthase (*entA*) aufweist.

2. Verfahren gemäß Anspruch 1, **dadurch gekennzeichnet, daß** die aus dem Organismus in ein wäßriges Permeat abgegebenen *trans*-Dihydroxy-Cyclohexadien-Carbonsäuren ohne zusätzlich vorgeschaltete Aufarbeitungsschritte aus diesem wäßrigen Permeat, bevorzugt durch Reaktivextraktion mittels eines kationischen Carriers oder die Adsorption/Desorption an einem Anionenaustauscher oder eine flüssig-flüssig-Extraktion isoliert werden, wobei enantiomerenreine *trans*-DCHC in Produktreinheiten von wenigstens 90% erhalten werden.

3. Verfahren gemäß einem der Ansprüche 1 oder 2, **dadurch gekennzeichnet, daß** *Escherichia coli* eingesetzt wird.

4. Verfahren gemäß einem der Ansprüche 1 bis 3, **dadurch gekennzeichnet, daß** für die chiralen *trans*-Dihydroxy-Cyclohexadien-Carbonsäuren eine Enantiomeren-Reinheit (ee) im Bereich von ≥ 99,9 % erreicht wird.

5. Verfahren gemäß einem der Ansprüche 1 bis 4, **dadurch gekennzeichnet, daß** bei der Reaktivextraktion als kationischer Carrier Ammoniumderivate, bevorzugt Ammoniumdervate mit sterisch anspruchsvollen Substituenten und/oder kationische Carrier aus der Gruppe der Cyclodextrin-Kationen, endrohedralkationische Fullerene, kationische geladene Kronenether und/oder Kryptanden eingesetzt werden.

6. Verfahren gemäß einem der Ansprüche 1 bis 5, **dadurch gekennzeichnet, daß** bei der Reaktivextraktion kationische Carrier in einer Konzentration im Bereich von 1-30 Vol.-%, bevorzugt 3-20 Vol.-%, besonders bevorzugt 5-15 Vol.-% bezogen auf das Kulturmedium eingesetzt werden.

7. Verfahren gemäß einem der Ansprüche 1 bis 6, **dadurch gekennzeichnet, daß** bei der Reaktivextraktion die Extraktionsleistung annähernd proportional zur eingesetzten Carrierkonzentration zunimmt.

8. Verfahren gemäß einem der Ansprüche 1 bis 7, **dadurch gekennzeichnet, daß** bei der Reaktivextraktion als organisches Lösungsmittel Sauerstoff-enthaltende Lösungsmittel mittlerer Kettenzahl, bevorzugt 2-Undecanon und/oder Diphenylether und/oder Butylbenzol und/oder 1-Octanol eingesetzt werden, wobei 1-Octanol besonders bevorzugt wird.

9. Verfahren gemäß einem der Ansprüche 1 bis 8, **dadurch gekennzeichnet, daß** bei der Reaktivextraktion als Reextraktionsmittel gesättigte anorganische Salzlösungen, bevorzugt mit Chlorid- und/oder Carbonat-Ionen, besonders bevorzugt in Form einer gesättigten Natriumchloridlösung, eingesetzt werden.

10. Verfahren gemäß einem der Ansprüche 1 bis 9, **dadurch gekennzeichnet, daß** bei der Reaktivextraktion die Reextraktionsleistung proportional zur Anionenkonzentration zunimmt und keine direkte Abhängigkeit vom pH-Wert vorliegt.

11. Verfahren gemäß einem der Ansprüche 1 bis 10, **dadurch gekennzeichnet, daß** die Extraktion der enantiomerenreinen *trans*-Dihydroxy-Cyclohexadien-Carbonsäure aus der Salzfracht nahezu quantitativ bei saurem pH-Wert, bevorzugt im Bereich von pH 0 bis pH 5, besonders bevorzugt bei pH 3, mit organischen Lösungsmitteln, bevorzugt mittelkettigen Alkoholen, flüchtigen Carbonsäureestem, Ketonen und/oder funktionalisierten Aromaten, besonders bevorzugt 1-Butanol, Essigester, Aceton und/oder Diphenylether, erfolgt.

12. Verfahren gemäß einem der Ansprüche 1 bis 4, **dadurch gekennzeichnet, daß** die Adsorption/Desorption in einem Fließbett oder Festbett, ggf. mit vorhergehender Abtrennung von Feststoffen erfolgt.

13. Genetisch veränderter Organismus zur verbesserten Herstellung von *trans*-Dihydroxy-Cyclohexadien-Carbonsäuren in enantiomerenreiner Form ohne nachweisbare Verunreinigungen mit Chorismat, **dadurch gekennzeichnet, daß** er ein genetisch veränderten Organismus der Gattung der Enterobakterien ist, der höchstens den Sicherheitsanforderungen der Risikogruppe 1 zugeordnet ist, welcher über einen Aromatenstoffwechsel verfügt und im Vergleich zu einem entsprechenden nicht genetisch veränderten Organismus
a) eine erhöhte Aktivität der Isochorismatsynthase (EntC) und Isochorismatase (EntB) im Vergleich zu *E. coli*-Stamm AN193, hervorgerufen durch Veränderungen auf transkriptionaler Ebene, und keine Aktivität der 2,3-Dihydroxybenzoatsynthase (EntA) durch eine vollständige Inaktivierung des Gens kodierend für die 2,3-Dihydroxybenzoatsynthase (*entA*) aufweist oder
b) eine erhöhte Aktivität der Isochorismatase (EntB), hervorgerufen durch Veränderungen auf transkriptionaler Ebene und eine erniedrigte Aktivität der Isochorismatsynthase (EntC), hervorgerufen durch Veränderungen auf transkriptionaler Ebene oder translationaler Ebene mittels antisense-RNA im Vergleich zu *E. coli*-Stamm AN193, und keine Aktivität der 2,3-Dihydroxybenzoatsynthase (EntA) durch eine vollständige Inaktivierung des Gens kodierend für die 2,3-Dihydroxybenzoatsynthase (*entA*) aufweist.

14. Genetisch veränderter Organismus gemäß Anspruch 13, **dadurch gekennzeichnet, daß** das *entA*-Gen vollständig oder partiell, bevorzugt über einem Bereich von wenigstens 1-99% und bevorzugt 5-90% deletiert ist.

15. Genetisch veränderter Organismus gemäß einem der Ansprüche 13 oder 14, **dadurch gekennzeichnet, daß** er durch eine verstärkte Expression des *entC*-Gens hervorgerufen durch Veränderungen auf transkriptionaler Ebene im Vergleich zu *E. coli*-Stamm AN193 vermehrt (5S,6S)-Dihydroxy-cyclohexa-1,3-diencarbonsäure bildet.

16. Genetisch veränderter Organismus gemäß einem der Ansprüche 13 bis 15, **dadurch gekennzeichnet, daß** er durch eine verminderte Expression des *entC*-Gens hervorgerufen durch Veränderungen auf transkriptionaler Ebene oder translationaler Ebene mittels antisense-RNA im Vergleich zu *E. coli*-Stamm AN193 vermehrt (3*R*,4*R*)-Dihydroxy-cyclohexa1,5-diencarbonsäure bildet.

17. Genetisch veränderter Organismus gemäß einem der Ansprüche 13 bis 16, **dadurch gekennzeichnet, daß** er ein Genkonstrukt und/oder einen Vektor enthält, das/der wenigstens einen, bevorzugt regulierbaren, Promotor aufweist, der mit dem *entC*-Strukturgen operativ verknüpft ist.

18. Genetisch veränderter Organismus gemäß einem der Ansprüche 13 bis 17, **dadurch gekennzeichnet, daß** er ein Genkonstrukt und/oder einen Vektor enthält, das/der wenigstens einen, bevorzugt regulierbaren, Promotor aufweist, der mit dem *entC*-Strukturgen in "antisense"-Orientierung operativ verknüpft ist.

## Claims

1. Process for the improved preparation of *trans*-dihydroxycyclohexadienecarboxylic acids and/or their secondary products, **characterized in that** the *trans*-dihydroxycyclohexadienecarboxylic acids are synthesized in enantiomerically pure form and without any detectable contamination with chorismate, proceeding from renewable carbon sources by culturing at least one genetically altered organism from the genus Enterobacteria which is assigned at most to the risk group 1 safety requirements, which has an aromatic metabolism and, as compared with an organism which is not correspondingly altered genetically,
a) exhibits an increased activity of isochorismate synthase (EntC) and isochorismatase (EntB) as compared with E. coli strain AN193, brought about by changes at transcriptional level, and does not exhibit activity of 2,3-dihydroxybenzoate synthase (EntA) as a result of the gene encoding 2,3-dihydroxybenzoate synthase (entA) being completely inactivated, or
b) exhibits an increased activity of isochorismatase (EntB), brought about by changes at transcriptional level, and a decreased activity of isochorismate synthase (EntC), brought about by changes at transcriptional level or translational level by means of antisense RNA, as compared with E. coli strain AN193, and does not exhibit activity of 2,3-dihydroxybenzoate synthase (EntA) as a result of the gene encoding 2,3-dihydroxybenzoate synthase (entA) being completely inactivated.

2. Process according to Claim 1, **characterized in that** the *trans*-dihydroxycyclohexandienecarboxylic acids, which are released from the organism into an aqueous permeate, are isolated from this aqueous permeate without any additional preliminary working-up steps, preferably by means of reactive extraction using a cationic carrier or by means of adsorption/desorption on an anion exchanger or by means of a liquid-liquid extraction, with enantiomerically pure *trans*-DCHCs being obtained in product purities of at least 90%.

3. Process according to either of Claims 1 and 2, **characterized in that** use is made of *Escherichia coli*.

4. Process according to one of Claims 1 to 3, **characterized in that** an enantiomeric purity (ee) in the region of ≥ 99.9% is achieved for the chiral *trans*-dihydroxycyclohexadienecarboxylic acids.

5. Process according to one of Claims 1 to 4, **characterized in that** ammonium derivatives, preferably ammonium derivatives having sterically exacting substituents, and/or cationic carriers from the group of cyclodextrin cations, endohedral-cationic fullerenes, cationic charged crown ethers and/or cryptands are employed as cationic carriers in the reactive extraction.

6. Process according to one of Claims 1 to 5, **characterized in that** cationic carriers are used in the reactive extraction at a concentration in the range of 1-30% by volume, preferably 3-20% by volume, particularly preferably 5-15% by volume, based on the culture medium.

7. Process according to one of Claims 1 to 6, **characterized in that**, in the reactive extraction, the extraction efficiency increases approximately in proportion to the carrier concentration employed.

8. Process according to one of Claims 1 to 7, **characterized in that** oxygen-containing solvents of average chain number, preferably 2-undecanone and/or diphenyl ether and/or butylbenzene and/or 1-octanol, are employed as organic solvent in the reactive extraction, with 1-octanol being particularly preferred.

9. Process according to one of Claims 1 to 8, **characterized in that** saturated inorganic salt solutions, preferably containing chloride and/or carbonate ions, particularly preferably in the form of a saturated sodium chloride solution, are employed as reextraction agents in the reactive extraction.

10. Process according to one of Claims 1 to 9, **characterized in that**, in the reactive extraction, the reextraction efficiency increases in proportion to the anion concentration and there is no direct dependence on the pH.

11. Process according to one of Claims 1 to 10, **characterized in that** the extraction of the enantiomerically pure *trans*-dihydroxycyclohexadienecarboxylic acid from the saline solution takes place virtually quantitatively at an acid pH, preferably in the range from pH 0 to pH 5, particularly preferably at pH 3, using organic solvents, preferably medium-chain alcohols, volatile carboxylic esters, ketones and/or functionalized aromatic compounds, particularly preferably 1-butanol, ethyl acetate, acetone and/or diphenyl ether.

12. Process according to one of Claims 1 to 4, **characterized in that** the adsorption/desorption takes place in a fluidized bed or fixed bed, where appropriate with solids being previously separated off.

13. Genetically altered organism for the improved preparation of *trans*-dihydroxycyclohexadienecarboxylic acids in enantiomerically pure form without any detectable contamination with chorismate, **characterized in that** it is a genetically altered organism from the genus Enterobacteria which is assigned to at most the risk group 1 safety requirements, which has an aromatic metabolism and, as compared with a corresponding organism which is not genetically altered,
a) exhibits an increased activity of isochorismate synthase (EntC) and isochorismatase (EntB) as compared with E. coli strain AN193, brought about by changes at transcriptional level, and does not exhibit activity of 2,3-dihydroxybenzoate synthase (EntA) as a result of the gene encoding 2,3-dihydroxybenzoate synthase (entA) being completely inactivated, or
b) exhibits an increased activity of isochorismatase (EntB), brought about by changes at transcriptional level, and a decreased activity of isochorismate synthase (EntC), brought about by changes at transcriptional level or translational level by means of antisense RNA, as compared with E. coli strain AN193, and does not exhibit activity of 2,3-dihydroxybenzoate synthase (EntA) as a result of the gene encoding 2,3-dihydroxybenzoate synthase (entA) being completely inactivated.

14. Genetically altered organism according to Claim 13, **characterized in that** the *ent*A gene is completely or partially deleted, preferably over a range of at least 1-99%, preferably 5-90%.

15. Genetically altered organism according to either of Claims 13 and 14, **characterized in that** it forms (5*S*,6*S*)-dihydroxycyclohexa-1,3-diene-carboxylic acid to an increased extent as a result of the expression of the *entC* gene being increased, brought about by changes at transcriptional level, as compared with E.coli strain AN193 .

16. Genetically altered organism according to one of Claims 13 to 15, **characterized in that** it forms (3*R*,4*R*)-dihydroxycyclohexa-1,5-dienecarboxylic acid to an increased extent as a result of the expression of *ent*C gene being decreased, brought about by changes at transcriptional level or translational level by means of antisense RNA, as compared with E.coli strain AN193.

17. Genetically altered organism according to one of Claims 13 to 16, **characterized in that** it contains a gene construct and/or a vector which has at least one promoter, which is preferably regulable and which is operatively linked to the *entC* structural gene.

18. Genetically altered organism according to one of Claims 13 to 17, **characterized in that** it contains a gene construct and/or vector which has at least one promoter, which is preferably regulable and which is operatively linked to the *entC* structural gene in the antisense orientation.

## Revendications

1. Procédé de préparation améliorée d'acides *trans*-dihydroxycyclohexadiènecarboxyliques et/ou de leurs produits secondaires, **caractérisé en ce que** la synthèse des acides *trans*-dihydroxycyclohexadiènecarboxyliques est réalisée sous forme énantiomériquement pure sans impuretés détectables avec du chorismate, en partant de sources de carbone renouvelables par culture d'au moins un organisme génétiquement modifié de l'espèce des entérobactéries, qui est affecté au plus aux exigences de sécurité du groupe de risques 1, lequel dispose d'un métabolisme des substances aromatiques et, par comparaison à un organisme non génétiquement modifié correspondant
a) présente une activité accrue de l'isochorismate synthase (EntC) et de l'isochorismatase (EntB) par comparaison à la souche de *E. coli* AN193, causée par des transformations par rapport au plan de transcription, et ne présente aucune activité de la 2,3-dihydroxybenzoate synthase (EntA) par une inactivation totale du gène codant pour la 2,3-dihydroxybenzoate synthase (*entA*), ou
b) présente une activité accrue de l'isochorismatase (EntB), causée par des transformations par rapport au plan de transcription, et une activité réduite de l'isochorismate synthase (EntC), causée par des transformations par rapport au plan de transcription ou au plan de translation au moyen d'ARN antisens, par comparaison à la souche de *E. coli* AN193, et ne présente aucune activité de la 2,3-dihydroxybenzoate synthase (EntA) par une inactivation totale du gène codant pour la 2,3-dihydroxybenzoate synthase (*entA*).

2. Procédé selon la revendication 1, **caractérisé en ce que** les acides *trans*-dihydroxycyclohexadiènecarboxyliques libérés de l'organisme dans un perméat aqueux sont isolés de ce perméat aqueux sans étapes de traitement préliminaires supplémentaires, de préférence par extraction réactive au moyen d'un support cationique ou par adsorption/désorption sur un échangeur d'anions ou par une extraction liquide-liquide, des *trans*-DCHC énantiomériquement purs étant obtenus avec des puretés de produit d'au moins 90 %.

3. Procédé selon l'une quelconque des revendications 1 ou 2, **caractérisé en ce que** l'on utilise *Escherichia coli*.

4. Procédé selon l'une quelconque des revendications 1 à 3, **caractérisé en ce qu'**une pureté énantiomérique (ee) dans la plage ≥ 99,9 % est obtenue pour les acides *trans*-dihydroxycyclohexadiènecarboxyliques chiraux.

5. Procédé selon l'une quelconque des revendications 1 à 4, **caractérisé en ce que** lors de la réaction extractive, des dérivés d'ammonium, de préférence des dérivés d'ammonium renfermant des substituants stériquement astreignants et/ou des supports cationiques parmi le groupe des cations de cyclodextrine, des fullerènes cationiques endoédriques, des éther-couronnes chargés cationiques et/ou des cryptands sont utilisés en tant que support cationique.

6. Procédé selon l'une quelconque des revendications 1 à 5, **caractérisé en ce que** lors de l'extraction réactive, on utilise des supports cationiques en une concentration dans la plage de 1 à 30 % en volume, de préférence de 3 à 20 % en volume, particulièrement préférablement de 5 à 15 % en volume, par rapport au milieu de culture.

7. Procédé selon l'une quelconque des revendications 1 à 6, **caractérisé en ce que** lors de l'extraction réactive, l'efficacité d'extraction augmente approximativement proportionnellement à la concentration en support utilisée.

8. Procédé selon l'une quelconque des revendications 1 à 7, **caractérisé en ce que** lors de l'extraction réactive, on utilise des solvants oxygénés présentant un nombre de chaînes moyen, de préférence la 2-undécanone et/ou l'éther diphénylique et/ou le butylbenzène et/ou le 1-octanol, en tant que solvant organique, le 1-octanol étant particulièrement préféré.

9. Procédé selon l'une quelconque des revendications 1 à 8, **caractérisé en ce que** lors de l'extraction réactive, on utilise des solutions de sels inorganiques saturées, de préférence avec des ions chlorure et/ou carbonate, particulièrement préférablement sous forme d'une solution saturée de chlorure de sodium, en tant qu'agent de réextraction.

10. Procédé selon l'une quelconque des revendications 1 à 9, **caractérisé en ce que** lors de l'extraction réactive, l'efficacité de réextraction augmente proportionnellement à la concentration en anions et il n'existe pas de dépendance directe au pH.

11. Procédé selon l'une quelconque des revendications 1 à 10, **caractérisé en ce que** l'extraction de l'acide *trans*-dihydroxycyclohexadiènecarboxylique énantiomériquement pur à partir de la charge saline est réalisée pratiquement quantitativement à un pH acide, de préférence dans la plage de pH 0 à pH 5, particulièrement préférablement à pH 3, avec des solvants organiques, de préférence des alcools à chaîne moyenne, des esters carboxyliques, des cétones et/ou des composés aromatiques fonctionnalisés volatils, particulièrement préférablement le 1-butanol, l'acétate d'éthyle, l'acétone et/ou l'éther diphénylique.

12. Procédé selon l'une quelconque des revendications 1 à 4, **caractérisé en ce que** l'adsorption/désorption est réalisée dans un lit fluidisé ou un lit fixe, le cas échéant avec séparation préalable des matières solides.

13. Organisme génétiquement modifié pour la préparation améliorée d'acides *trans*-dihydroxycyclohexadiènecarboxyliques sous forme énantiomériquement pure sans impuretés détectables avec du chorismate, **caractérisé en ce qu'**il est un organisme génétiquement modifié de l'espèce des entérobactéries, qui est affecté au plus aux exigences de sécurité du groupe de risques 1, lequel dispose d'un métabolisme des substances aromatiques et, par comparaison à un organisme non génétiquement modifié correspondant
a) présente une activité accrue de l'isochorismate synthase (EntC) et de l'isochorismatase (EntB) par comparaison à la souche de *E. coli* AN193, causée par des transformations par rapport au plan de transcription, et ne présente aucune activité de la 2,3-dihydroxybenzoate synthase (EntA) par une inactivation totale du gène codant pour la 2,3-dihydroxybenzoate synthase (*entA*), ou
b) présente une activité accrue de l'isochorismatase (EntB), causée par des transformations par rapport au plan de transcription, et une activité réduite de l'isochorismate synthase (EntC), causée par des transformations par rapport au plan de transcription ou au plan de translation au moyen d'ARN antisens, par comparaison à la souche de *E. coli* AN193, et ne présente aucune activité de la 2,3-dihydroxybenzoate synthase (EntA) par une inactivation totale du gène codant pour la 2,3-dihydroxybenzoate synthase (*entA*).

14. Organisme génétiquement modifié selon la revendication 13, **caractérisé en ce que** le gène *entA* est totalement ou partiellement supprimé, de préférence sur une plage d'au moins 1 à 99 % et de préférence de 5 à 90 %.

15. Organisme génétiquement modifié selon l'une quelconque des revendications 13 ou 14, **caractérisé en ce qu'**il forme, dans une mesure accrue, de l'acide (5S,6S)-dihydroxycyclohexa-1,3-diènecarboxylique par une expression renforcée du gène *entC* causée par des transformations par rapport au plan de transcription, par comparaison à la souche de *E. coli* AN193.

16. Organisme génétiquement modifié selon l'une quelconque des revendications 13 à 15, **caractérisé en ce qu'**il forme, dans une mesure accrue, de l'acide (3R,4R)-dihydroxycyclohexa-1,5-diènecarboxylique par une expression réduite du gène *entC* causée par des transformations par rapport au plan de transcription ou au plan de translation au moyen d'ARN antisens par comparaison à la souche de *E. coli* AN193.

17. Organisme génétiquement modifié selon l'une quelconque des revendications 13 à 16, **caractérisé en ce qu'**il contient un produit de recombinaison génique et/ou un vecteur qui présente au moins un promoteur de préférence régulable, qui est lié fonctionnellement au gène structural *entC*.

18. Organisme génétiquement modifié selon l'une quelconque des revendications 13 à 17, **caractérisé en ce qu'**il contient un produit de recombinaison génique et/ou un vecteur qui présente au moins un promoteur de préférence régulable, qui est lié fonctionnellement au gène structural *entC*, en orientation « antisens ».


### SEQUENCE LISTING

<110> Forschungszentrum Juelich GmbH
<120> Verfahren zur verbesserten Herstellung und Isolierung von Trans-Dihydroxy-Cyclohexadien-Carbonsäuren und/oder deren Folgeprodukte sowie ein dazu geeigneter genetisch veränderter Organismus
<130> 1
<140> PCT/EP01/09980
   <141> 2001-08-30
<150> DE 10042535.6
   <151> 2000-08-30
<160> 4
<170> PatentIn Ver. 2.1
<210> 1
   <211> 24
   <212> DNA
   <213> Artificial Sequence
<220>
   <223> Description of Artificial Sequence:PCR primer entB3'-5'
<400> 1
   gggctgcaga catttttacc gctg 24
<210> 2
   <211> 26
   <212> DNA
   <213> Artificial Sequence
<220>
   <223> Description of Artificial Sequence:PCR primer entB5'-3'
<400> 2
   tatggatcca cgcgcatcag cctgaa 26
<210> 3
   <211> 23
   <212> DNA
   <213> Artificial Sequence
<220>
   <223> Description of Artificial Sequence:PCR primer entC3'-5'
<400> 3
   tgcggatcct cgctccttat tgc 23
<210> 4
   <211> 24
   <212> DNA
   <213> Artificial Sequence
<220>
   <223> Description of Artificial Sequence:PCR primer entC5'-3'
<400> 4
   ggcgagctca ttattaaagc cttt 24